(19)

**Europäisches Patentamt**

**European Patent Office**

**Office européen des brevets**

(11) **EP 4 610 709 A1**

(12) **EUROPEAN PATENT APPLICATION**
published in accordance with Art. 153(4) EPC

(43) Date of publication:
**03.09.2025 Bulletin 2025/36**

(21) Application number: **22963064.5**

(22) Date of filing: **27.10.2022**

(51) International Patent Classification (IPC):
**G02B 21/02** (2006.01)   **C12Q 1/6809** (2018.01)
**C12Q 1/6869** (2018.01)

(52) Cooperative Patent Classification (CPC):
**C12Q 1/6809; C12Q 1/6869; G02B 21/02**

(86) International application number:
**PCT/CN2022/127815**

(87) International publication number:
**WO 2024/087080 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **MGI Tech Co., Ltd.**
**Shenzhen, Guangdong 518083 (CN)**

(72) Inventors:
• HUANG, Yi
  Shenzhen, Guangdong 518083 (CN)
• YANG, Bin
  Shenzhen, Guangdong 518083 (CN)
• JIANG, Heming
  Shenzhen, Guangdong 518083 (CN)

(74) Representative: **Bryn Aarflot AS**
**Patent**
**Stortingsgata 8**
**0161 Oslo (NO)**

(54) **IMMERSION OBJECTIVE, OPTICAL SYSTEM AND DETECTION METHOD**

(57)    An immersion objective, an optical system and a detection method. The immersion objective sequentially comprises, in the direction from an object side (S) to an image side, a first lens group (G1) having a positive focal power, at least one group of lens groups (G5, G6) having a negative focal power, and an immersion medium (m) which is located on the object side (S) of the first lens group (G1) and immerses at least part of the first lens group (G1), wherein the working distance $S_0$ of the immersion medium (m) between the first lens group (G1) and the object side (S) and the focal length $F_{obj}$ of the immersion objective satisfy the conditional expression $0 \leq S_0/F_{obj} \leq 0.16$. By means of the cooperation of a plurality of groups of lenses, an immersion objective has a relatively small size and a relatively low mass within a relatively compact size range, and a large field of view, a high resolution and a low distortion are achieved, thereby improving the optical performance of the immersion objective.

**FIG. 1E**

EP 4 610 709 A1

**Description**

**FIELD**

The present disclosure relates to the technical field of optics, in particular to an immersion objective, an optical system and a detection method.

**BACKGROUND**

A large field-of-view apochromatic objective that approaches diffraction-limited imaging quality is typically provided with a dozen or so of lenses or tens of lenses to balance various aberrations introduced by a large field of view and a high numerical aperture (NA), resulting in a complex structure of the objective, thus increasing the difficulty of performing optical design under a limitation of specific size and weight. Additionally, the manufacturing and assembly of an objective impose stringent requirements on element fabrication tolerances, objective assembly precision, and system detection precision that reach the highest level in the industry, which necessitates expensive devices for manufacturing, assembly and detecting, or devices for retrofitting, as well as highly experienced engineers and operators.

A field of view of a microscope refers to an object-side area that can be obtained within a unit of time. For a to-be-detected object with a determined size, a larger object-space field of view indicates a smaller number of detections for completely performing the detection of the object, thereby saving time and reducing operations. The numerical aperture and working wavelength of a microscope determine the resolution of the microscope. The numerical aperture $NA=n*\sin(\theta)$, where $n$ represents a refractive index of an object-space medium and $\theta$ represents an object-space semi-aperture angle of the objective. The numerical aperture can be increased by increasing the object-space semi-aperture angle of the objective or the refractive index of the object-space medium, thereby improving the refractive index.

For the conventional commercial microscopes, very few of them are provided both with a large field of view and a high numerical aperture, as well as flat-field apochromatism, where the product of the object-space field of view (in mm) and the numerical aperture is typically less than 0.75, which does not meet practical requirements.

**SUMMARY**

In view of the above, objectives of the present disclosure are to provide an immersion objective, an optical system, and a detection method, to achieve a large field of view and a high numerical aperture, so as to obtain a high-quality immersion objective and optical system.

In an embodiment of the present disclosure, an immersion objective is provided. The immersion objective includes: sequentially along a direction from an object space to an image space, a first lens group with positive focal power, at least one lens group with negative focal power and an immersion medium. The immersion medium is located on an object side of the first lens group, and at least part of the first lens group is immersed in the immersion medium. A working distance $S_0$ of the immersion medium located between the first lens group and the object space and a focal length $F_{obj}$ of the immersion objective satisfy a conditional expression:

$$0<S_0/F_{obj}\leq0.16$$

In an embodiment, the at least one lens group with negative focal power includes: sequentially along the direction from the object space to the image space, a third lens with positive focal power, a second lens group with positive focal power, a third lens group with positive focal power, a fourth lens group with positive focal power, a fifth lens group with negative focal power and a sixth lens group with negative focal power. It should be understood that, for those skilled in the art, the number of lenses or lens groups can be reduced or increased through adjusting surface types of lenses and relative positions between lenses. Adjustments made based on the concept of the present disclosure and without creative efforts are also within the protection scope of the present disclosure.

In an embodiment, the immersion medium includes a first medium with a first refractive index $n_{00}$ and a second medium with a second refractive index $n_{01}$; the first medium is located between the second medium and the first lens group and has a thickness $d_1$, and the second medium has a thickness $d2$, where $S_0=d_1+d_2$; and the immersion objective further satisfies a conditional expression:

$$0<(n_{00}d_1+n_{01}d_2)/F_{obj}\leq0.20$$

In an embodiment, $n_{01}$ is greater than $n_{00}$.
In an embodiment, the first medium is a liquid and the second medium is a solid.

In an embodiment, the first medium is water and the second medium is a glass plate.

In an embodiment, the first lens group, the second lens group, the fourth lens group, and the sixth lens group are doublet cemented lens groups, the fifth lens group includes a doublet cemented lens group, and the second lens group and the fourth lens group are symmetrically arranged with respect to the third lens group.

In an embodiment, the first lens group includes a first lens and a second lens that are sequentially cemented along the direction from the object space to the image space, an object-side surface of the first lens is a plane surface, an image-side surface of the first lens is a convex surface, an image-side surface of the second lens is a convex surface, and a ratio of a focal length of the first lens group to the focal length of the immersion objective is in a range of [3.39, 4.4].

In an embodiment, the third lens is a meniscus lens, a ratio of a radius of curvature of an image-side surface of the third lens to a first distance is in a range of (-0.8, 1.0], the first distance is an axial distance between the image-side surface of the third lens and an object plane, and a ratio of a focal length of the third lens and the focal length of the immersion objective is in a range of (-6.6, 7.27].

In an embodiment, the second lens group includes a fourth lens and a fifth lens that are sequentially cemented along the direction from the object space to the image space, and an absolute value of a difference between a refractive index of the fourth lens and a refractive index of the fifth lens is greater than 0.2.

In an embodiment, the third lens group is a triplet cemented lens group, and the third lens group includes a sixth lens, a seventh lens, and an eighth lens that are sequentially cemented along the direction from the object space to the image space.

In an embodiment, the fourth lens group includes a ninth lens and a tenth lens that are sequentially cemented along the direction from the object space to the image space, and a ratio of a focal length of the fourth lens group to the focal length of the immersion objective is less than 21.10.

In an embodiment, the immersion objective further includes an aperture stop.

In an embodiment, the sixth lens group includes a fourteenth lens and a fifteenth lens that are sequentially cemented along the direction from the object space to the image space, and an ratio of a focal length of the sixth lens group to the focal length of the immersion objective is in a range of (-21.51, -17.4].

In an embodiment, the doublet cemented lens group included in the fifth lens group includes an eleventh lens and a twelfth lens that are sequentially cemented along the direction from the object space to the image space. The fifth lens group further includes a thirteenth lens, and the thirteenth lens is located between the twelfth lens and the sixth lens group.

In an embodiment of the present disclosure, an optical system is provided. The optical system includes the immersion objective and an imaging device. The imaging device is located on an image side of the immersion objective and works in conjunction with the immersion objective.

In an embodiment, the optical system further includes a tube lens located between the immersion objective and the imaging device.

In an embodiment, the optical system further includes a light source for stimulating a to-be-detected sample to generate a stimulated light. The immersion objective is configured to direct a light beam generated by the light source to the to-be-detected sample and transmit the stimulated light. The imaging device includes a time delay integration camera. The time delay integration camera is configured to record the stimulated light and image the to-be-detected sample.

In an embodiment, the optical system further includes a light source shaping system located between the light source and the immersion objective. The light source shaping system is configured to shape the light beam generated by the light source.

In an embodiment, the optical system further includes a compensation lens. The compensation lens is removably arranged at an optical path between the immersion objective and the imaging device.

In an embodiment of the present disclosure, a detection method is provided. The method includes: detecting a to-be-detected sample by using the optical system.

In an embodiment, the to-be-detected sample includes at least two sample detection surfaces spaced apart from each other along an optical axis direction of the immersion objective. The detecting a to-be-detected sample by using the optical system includes:

adjusting an immersion depth of the immersion objective in the immersion medium to detect different sample detection surfaces.

In an embodiment of the present disclosure, a nucleic acid detection system is further provided. The nucleic acid detection system includes sequencing chip and an optical system. The sequencing chip is configured to support a to-be-detected sample. The optical system includes an objective and an immersion medium arranged between the objective and the to-be-detected sample. An end of the objective close to the to-be-detected sample is immersed in the immersion medium. A working distance $S_0$ of the immersion medium between the objective and the to-be-detected sample and a focal length $F_{obj}$ of the objective satisfy a conditional expression:

$$0 < S_0/F_{obj} \leq 0.16$$

In an embodiment, the objective includes, sequentially along a direction from an object space to an image space, a first lens group with positive focal power and at least one lens group with negative focal power. The immersion medium is located on an object side of the first lens group, and at least part of the first lens group is immersed in the immersion medium.

In an embodiment, the at least one lens group with negative focal power includes: sequentially along the direction from the object space to the image space, a third lens with positive focal power, a second lens group with positive focal power, a third lens group with positive focal power, a fourth lens group with positive focal power, a fifth lens group with negative focal power, and a sixth lens group with negative focal power. It should be understood that, for those skilled in the art, the number of lenses or lens groups can be reduced or increased through adjusting surface types of lenses and relative positions between lenses. Adjustments made based on the concept of the present disclosure and without creative efforts are also within the protection scope of the present disclosure.

In an embodiment, the optical system further an imaging device, located on an image side of the objective and works in conjunction with the objective; and a tube lens, located between the objective and the imaging device.

In an embodiment, the immersion medium includes a coverslip covering the to-be-detected sample and an immersion liquid arranged on a side of the coverslip away from the to-be-detected sample. A refractive index of the immersion liquid is represented by $n_{00}$, a refractive index of the coverslip is represented by $n_{01}$, a working distance of the immersion liquid on an optical path of the objective is represented by $d_1$, and a thickness of the coverslip is represented by $d_2$, where $S_0 = d_1 + d_2$. The immersion objective further satisfies a conditional expression:

$$0 < (n_{00}d_1 + n_{01}d_2)/F_{obj} \leq 0.20$$

In an embodiment, the immersion liquid is water.

In an embodiment, the sequencing chip includes a slide spaced apart from a coverslip. The to-be-detected sample includes a first detection sample fixed to the slide and a second detection sample opposite to and spaced apart from the first detection sample and fixed to the coverslip.

In an embodiment of the present disclosure, a biochemical detection method is provided. The biochemical detection method includes: loading a to-be-detected sample into a lane of a chip flow cell; and loading a reagent with multiple different reaction components into the lane of the chip flow cell to perform a biochemical reaction between the to-be-detected sample and the reagent.

The reaction components include at least one of: a sample generation component and a sample analysis component.

In an embodiment, the biochemical reaction includes generating a sample in the lane of the chip flow cell, which includes: directing different sample generation components to flow into the lane and controlling reaction conditions in the lane to generate the sample.

The biochemical reaction includes analyzing the sample in the lane, which includes: directing the sample analysis component to flow into the lane, where the sample analysis component reacts with the sample to generate a detectable signal.

The biochemical detection method further includes identifying the detectable signal using an optical system. The optical system includes an objective and an immersion medium arranged between the objective and the to-be-detected sample, and a working distance $S_0$ of the immersion medium located between the first lens group and the object space and a focal length $F_{obj}$ of the immersion objective satisfy a conditional expression:

$$0 < S_0/F_{obj} \leq 0.16$$

In an embodiment, the objective includes, sequentially along a direction from an object space to an image space, a first lens group with positive focal power, a third lens with positive focal power, and at least one lens group with negative focal power. The immersion medium is located on an object side of the first lens group, and at least part of the first lens group is immersed in the immersion medium.

In an embodiment, the objective includes: sequentially along a direction from an object space to an image space: a first lens group, a third lens with positive focal power, a second lens group with positive focal power, a third lens group with positive focal power, a fourth lens group with positive focal power, a fifth lens group with negative focal power, and a sixth lens group with negative focal power.

It should be understood that, for those skilled in the art, the number of lenses or lens groups can be reduced or increased through adjusting surface types of lenses and relative positions between lenses. Adjustments made based on the concept of the present disclosure and without creative efforts are also within the protection scope of the present disclosure.

In an embodiment, the biochemical reaction is a nucleic acid sequencing reaction and the to-be-detected sample is a nucleic acid sequencing library.

In an embodiment, the detectable signal is an optical signal.

In an embodiment, the to-be-detected sample is a tissue sample and the biochemical reaction is a specific binding reaction.

An immersion objective, an optical system, and a detection method are provided according to embodiments of the present disclosure. The immersion objective includes: sequentially along a direction from an object space to an image space, a first lens group with positive focal power, at least one lens group with negative focal power and an immersion medium. The immersion medium is located on an object side of the first lens group, and at least part of the first lens group is immersed in the immersion medium. A working distance $S_0$ of the immersion medium located between the first lens group and the object space and a focal length $F_{obj}$ of the immersion objective satisfy the following conditional expression: $0<S_0/F_{obj}\leq0.16$. The combination of multiple lenses allows the immersion objective to achieve a relatively compact and lightweight design within a restricted size range, resulting in the immersion objective with a large field of view, high resolution, and minimal distortion, thus improving the optical performance of the immersion objective.

**BRIEF DESCRIPTION OF THE DRAWINGS**

For clearer illustration of the technical solutions according to embodiments of the present disclosure or the conventional technology, hereinafter briefly described are the drawings to be applied in the description of the embodiments of the present disclosure or the conventional technology. Apparently, the drawings in the following descriptions are only some embodiments of the present disclosure, and other drawings may be obtained by those skilled in the art based on the provided drawings without creative efforts.

FIG. 1A to FIG. 1E are structural schematic diagrams of an immersion objective according to an embodiment of the present disclosure;

FIG. 2 is a diagram of curves of wave-front error root mean square within two waveband ranges of an immersion objective according to an embodiment of the present disclosure;

FIG. 3 is a diagram of curves of wave-front errors varying with a focal plane according to an embodiment of the present disclosure;

FIG. 4 is a schematic diagram of a distortion feature of an immersion objective according to an embodiment of the present disclosure;

FIG. 5 is a diagram of curves of wave-front error root mean square within two waveband ranges of an immersion objective according to another embodiment of the present disclosure;

FIG. 6 is a diagram of curves of wave-front errors varying with a focal plane according to another embodiment of the present disclosure;

FIG. 7 is a schematic diagram of a distortion feature of an immersion objective according to another embodiment of the present disclosure;

FIG. 8 is a diagram of curves of wave-front error root mean square within two waveband ranges of an immersion objective according to another embodiment of the present disclosure;

FIG. 9 is a diagram of curves of wave-front errors varying with a focal plane according to another embodiment of the present disclosure;

FIG. 10 is a schematic diagram of operation of an immersion objective according to an embodiment of the present disclosure;

FIG. 11 illustrates a detection result of an immersion objective according to an embodiment of the present disclosure;

FIG. 12 is a schematic diagram of an optical system according to an embodiment of the present disclosure;

FIG. 13 is a diagram of curves of wave-front error root mean square of the optical system according to an embodiment of the present disclosure; and

FIG. 14 is a schematic structural diagram of an optical system according to another embodiment of the present disclosure.

## DETAILED DESCRIPTION

In order to make the above objectives, features, and advantages of the present disclosure more apparent and understandable, specific implementations of the present disclosure are described in detail below in conjunction with the drawings.

Various specific details are set forth in following description to facilitate a full understanding of the present disclosure. The present disclosure may be implemented in a manner different from those described herein, and those skilled in the art may perform analogous promotion without departing from concepts of the present disclosure. Therefore, the present disclosure is not limited by the embodiments disclosed hereinafter.

The present disclosure is described in detail in conjunction with schematic diagrams. In following description, a cross-sectional view of a device structure may be partially enlarged not according to a general scale in order to facilitate illustration. The schematic diagrams are only examples and are not intended for limiting a protection scope of the present disclosure. In addition, three-dimensional spatial dimensions of length, width, and depth shall be configured in practice.

For the conventional commercial microscopes, very few of them are provided both with a large field of view and a high numerical aperture, where the product of an object-space field of view (in mm) and an numerical aperture is typically less than 0.75, which does not meet actual demands and restricts the development in related fields.

For example, a sequencer is an instrument capable of determining a base sequence, a type, and a quantity of DNA fragments. The sequencer is mainly applied to human genome sequencing, genetic diagnosis of human hereditary diseases, infectious diseases, and cancers, forensic paternity testing and individual identification, biopharmaceutical screening, cross breeding of plants and animals, and the like. The pursuit of maximum high throughput, high speed, and cost-effectiveness guides the development of next-generation sequencers. Achieving these objectives necessitates that sequencers possess both a high numerical aperture and a large field of view.

Resolution of a sequencer determines information density the sequencer can process per unit time. High resolution facilitates high throughput and speed. Thus, requirements are specified for the sequencer to have a high numerical aperture. When an object-space semi-aperture angle reaches its limit, increasing a refractive index of an object-space medium is a manner for improving resolution.

The cost of sequencing includes three parts: cost of instrument depreciation, cost of sequencing reagents, and cost of sequencing chips, among which the cost of sequencing reagents and the cost of sequencing chip constitute the majority of the total cost. Therefore, DNA nanoballs (DNB points) from which a larger amount of data can be detected on a sequencing chip of the same area, or the ability to generate a greater number of DNB points on a sequencing chip of a sufficiently small area (which minimizes reagent loss), can both effectively reduce the cost of sequencing. This further achieves the continuation of the More than Moore's Law in terms of technology and fosters explosive growth in the application of sequencing technologies across industries related to human health, animals, plants, microorganisms, and the like. A high resolution of the sequencer is also conducive to reducing the cost of sequencing chips and reagents. Therefore, improving the resolution of the sequencer is crucial for improving the throughput and speed of the sequencer and reducing the cost of consumables.

In addition, the conventional immersion objectives are generally utilized in high-magnification microscopes, having both a high numerical aperture and a high magnification. However, the object-space field of view remains extremely limited, for example, an immersion objective with an NA of 1.0 typically has an object-space field of view of less than 1mm. In a sequencing process, with a predetermined sequencing chip size, a predetermined camera frame rate, and a predetermined platform movement speed, a larger object-space field of view indicates fewer photographs or less time for capturing the entire chip. Consequently, immersion objectives with a small object-space field of view are unsuitable for sequencers. Furthermore, most commercial objectives are designed for visual observation and fail to achieve sharp image quality close to a diffraction limit in the entire imaging range.

It is a significant research focus in this field to achieve a microscopic imaging system with both a high numerical aperture and a large field of view, particularly in an objective provided with both a high numerical aperture and a large field of view.

Currently, optical systems of most commercial sequencers are provided with air objectives. Due to constraints imposed by optical design, alignment, tolerances and the like, an object-space semi-aperture angle has an upper limit, typically not exceeding 0.9. The conventional immersion objectives are excessively large in size, volume, and weight, increasing difficulty for platform design, overall system design and industrialization. Furthermore, with a required actual flow rate of the sequencer increases, the coverslip for biochip becomes thicker, increasing the working distance, thus increasing the difficulty of the design process. In addition, a fluorescence microscope system of a sequencer uses an image sensor for imaging, which requires strict field curvature correction.

In view of this, an immersion objective, an optical system, and a detection method are provided according to embodiments of the present disclosure. The immersion objective includes: sequentially along a direction from an object space to an image space, a first lens group with positive focal power, a third lens with positive focal power, a second lens group with positive focal power, a third lens group with positive focal power, a fourth lens group with positive focal power, a fifth lens group with negative focal power, a sixth lens group with negative focal power, and an immersion medium located on an object-side of the first lens group and immersing at least part of the first lens group. A working distance $S_0$ of the immersion medium located between the first lens group and the object space and a focal length $F_{obj}$ of the immersion objective satisfy the following conditional expression: $0<S_0/F_{obj}\leq0.16$. The combination of multiple lenses allows the immersion objective to achieve a relatively compact and lightweight design within a restricted size range, resulting in the immersion objective with a large field of view, high resolution, and minimal distortion, thus improving the optical performance of the immersion objective.

For better understanding technical solutions and technical effects of the present disclosure, hereinafter embodiments are described in detail in conjunction with the drawings.

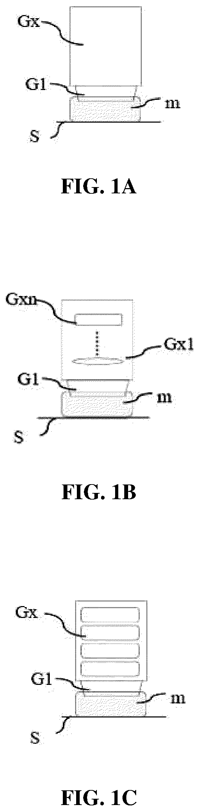

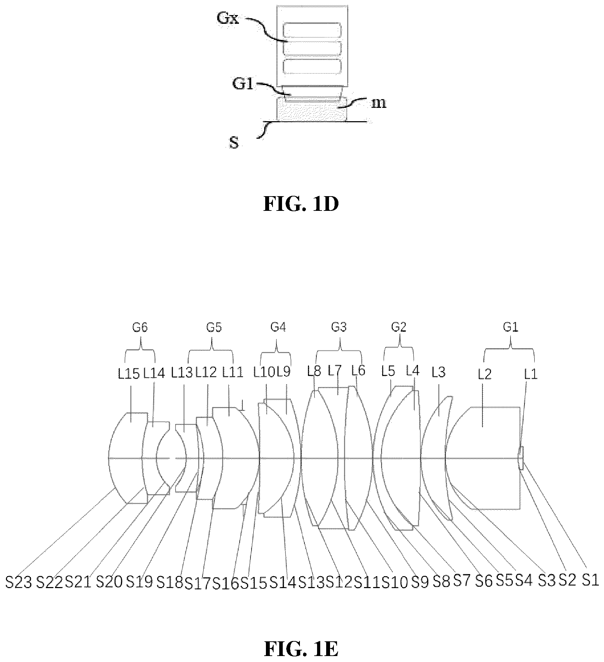

As shown in FIG. 1A to FIG. 1D, an immersion objective is provided according to the present disclosure. The immersion objective includes a first lens group G1, and a lens group Gx located, along an optical axis direction, on an image side of the first lens group G1. An immersion medium m is arranged between the lens group G1 and an object space S, and one end of the lens group G1 close to the object space is immersed in the immersion medium m. The number of lens groups Gx is not limited, and may be, for example, three and four as shown in FIG. 1C and FIG. 1D. The lens group Gx may be a single-piece lens (for example, Gx1 in FIG. 1B), a compound lens formed by multiple lenses (for example, Gxn in FIG. 1B), or the like. An optical signal of the object space S can be collected through adjusting immersion depth of the first lens group G1 in the immersion medium m. Depth of the immersion medium between the first lens group G1 and the object space S is the working distance $S_0$ of the immersion objective. The working distance $S_0$ and the focal length $F_{obj}$ of the objective lens satisfy the following condition:

$$0<S_0/F_{obj}\leq0.16$$

The working distance $S_0$ of the immersion objective may be altered by adjusting the immersion depth of the immersion objective in the immersion medium m, thereby meeting the above condition to achieve better image quality.

In an embodiment, reference is made to FIG. 1E, which is a schematic structural diagram of an immersion objective according to an embodiment of the present disclosure. The immersion objective according to the embodiment of the present disclosure includes, sequentially along the direction from the object space to the image space, the first lens group G1, the third lens L3, the second lens group G2, the third lens group G3, the fourth lens group G4, the fifth lens group G5, and the sixth lens group G6. The first lens group G1, the third lens L3, the second lens group G2, the third lens group G3, and the fourth lens group G4 have positive focal power, and the fifth lens group G5 and the sixth lens group G6 have negative focal power.

The immersion objective may further include the immersion medium located on the object side of the first lens group and immersing at least part of the first lens group. The working distance $S_0$ of the immersion medium (may also be referred to as an immersion working medium) located between the first lens group and the object space, and the focal length $F_{obj}$ of the immersion objective, satisfy the following condition:

$$0<S_0/F_{obj}\leq0.16$$

In an embodiment, the immersion medium includes a first medium and a second medium. The first medium has a first refractive index $n_{00}$ and the second medium has a second refractive index $n_{01}$. The first medium is located between the second medium and the first lens group and has a predetermined thickness $d_1$, and the second medium has a thickness $d_2$. The working distance $S_0$ of the immersion medium is expressed as $S_0=d_1+d_2$. The immersion objective further satisfies the following condition:

$$0<(n_{00}d_1+n_{01}d_2)/F_{obj}\leq0.20$$

In specific implementation, $n_{01}>n_{00}$. The first medium may be a liquid, and the second medium may be a solid. When the first medium is a liquid, it may also be referred to as an immersion liquid. The first medium may be water or another liquid. The second medium may be a glass plate. When the glass plate is placed over the to-be-detected sample, it is referred to as a coverslip.

The object space of the immersion objective may be provided with a sample solution, that is, the object side of the first lens group G0 includes the first medium, the second medium, and the sample solution in sequence, where the first medium is water and the second medium is a coverslip. The sample solution is provided with the to-be-detected sample, and the first medium may be provided with the to-be-detected sample. The plane where the to-be-detected sample is located is referred to as an object plane, and a side of the sample solution away from the second medium may be provided with a slide. The to-be-detected sample may be a biochemical chip or another detection sample. The refractive index of the first medium is close to the refractive index of the sample solution, which may range from 1.3 to 1.36. The thickness of the coverslip is represented by $d_2$, and the focal length of the immersion objective is represented by $f_{obj}$, where $0.06 < d_2/F_{obj} < 0.07$. An object-space numerical aperture $NA \leq 1.0$. An object-space semi-field of view height is represented by Ho, where $Ho/f_{obj} \leq 0.0625$, which is applicable to a microscopic objective with a magnification ranging from 10x to 100x. Taking a focal length of 10mm as an example, a maximum image-space semi-field of view height (maximum object-space field of view multiplied by magnification of a microscopic system) may reach 62.5mm. It should be understood that when the immersion medium is a liquid medium, and the sample solution of a to-be-detected object in the object space is in direct contact with the immersion medium, that is, the to-be-detected sample is an open structure provided with the sample solution, the immersion medium is preferably insoluble in the sample solution and has a density lower than the density of the sample solution.

The first lens group, the second lens group, the third lens group, the fourth lens group, the fifth lens group, and the sixth lens group may include a cemented lens group formed by multiple lenses. For example, the first lens group, the second lens group, the fourth lens group, and the sixth lens group are doublet cemented lens groups. The third lens group is a doublet cemented lens group or a triplet cemented lens group. The fifth lens group includes a doublet cemented lens group. The second lens group and the fourth lens group are arranged symmetrically with respect to the third lens group.

In an embodiment of the present disclosure, the first lens group G1 includes a first lens L1 and a second lens L2 that are sequentially cemented along the direction from the object space to the image space. The first lens L1 is closer to the object space than the second lens L2 is. An object-side surface of the first lens L1 is represented by S1. An image-side surface of the first lens L1 is cemented with an object-side surface of the second lens L2, and the cemented surface is represented by S2. An image-side surface of the second lens L2 is represented by S3. A ratio of a focal length of the first lens group to a focal length of the immersion objective is in a range of [3.39, 4.4], that is, the first lens group G1 satisfies the following condition: $3.39 \leq f_1/F_{obj} \leq 4.4$, where f1 represents the focal length of the first lens group G1 and $F_{obj}$ represents the focal length of the immersion objective.

A refractive index $n_1$ of the first lens L1 is less than 1.5 and may be close to the refractive index of water, reducing a spherical aberration. For example, the first lens L1 may be made of fused silica which has a refractive index close to the refractive index of water and has corrosion resistance. The first lens L1 may be a plano-convex lens, where an object-side surface S1 of the first lens L1 is a planar surface, and an image-side surface (that is, the cemented surface S2) of the first lens L1 is a convex surface. A refractive index of the second lens L2 may be greater than the refractive index of the first lens L1. For example, the refractive index $n_2$ of the second lens is greater than 1.8, balancing field curvature and simultaneously reducing chromatic aberrations. The image-side surface of the second lens L2 is a convex surface.

In an embodiment of the present disclosure, the third lens L3 may be a meniscus lens with relatively high positive focal power and works in conjunction with the first lens group G1 to provide positive focal power. An object-side surface of the third lens L3 is represented by S4, and an image-side surface of the third lens L3 is represented by S5. The ratio of a focal length of the third lens to the focal length of the immersion objective is in the range of (-6.6, 7.27]. The third lens L3 satisfies the following condition: $-0.8 < R_5/d_5 \leq 1.0$, and $6.6 < f_{31}/F_{obj} \leq 7.27$. $f_{31}$ represents the focal length of the second lens L3, $F_{obj}$ represents the focal length of the immersion objective, $R_5$ represents a radius of curvature of S5, and $d_5$ represents a first distance, where the first distance is an axial distance between the image-side surface S5 of the third lens and the object plane.

In an embodiment of the present disclosure, the second lens group G2 has a function of correcting chromatic aberrations in addition to further providing positive focal power. The second lens group G2 includes a fourth lens L4 and a fifth lens L5 that are sequentially cemented along the direction from the object space to the image space. The fourth lens L4 and the fifth lens L5 are cemented with each other. An object-side surface of the fourth lens L4 is represented by S6. An image-side surface of the fourth lens L4 is cemented with an object-side surface of the fifth lens L5, and the cemented surface is represented by S7. An image-side surface of the fifth lens L5 is represented by S8. An absolute value of a difference between a refractive index $n_4$ of the fourth lens L4 and a refractive index $n_5$ of the fifth lens L5 is greater than 0.2, that is, $|n_4 - n_5| > 0.2$. A focal length of the second lens group G2 is represented by $f_2$.

In an embodiment of the present disclosure, the third lens group G3 is configured to correct spherical aberrations, coma aberrations, and chromatic aberrations. The third lens group G3 includes at least two lens that are sequentially cemented along the direction from the object space to the image space. For example, the third lens group G3 includes a sixteenth lens and a seventeenth lens sequentially cemented along the direction from the object space to the image space. Alternatively, the third lens group G3 may include a sixth lens L6, a seventh lens L7, and an eighth lens L8 that are sequentially cemented along the direction from the object space to the image space. An object-side surface of the sixth lens L6 is represented by S9. An image-side surface of the sixth lens L6 is cemented with an object-side surface of the seventh lens L7, and the cemented surface is represented by S10. An image-side surface of the seventh lens L7 is cemented with an object-side surface of the eighth lens L8, and the cemented surface is represented by S11. An image-side surface of the eighth lens L8 is represented by S12. The ratio of a focal length of the third lens group to the focal length

**EP 4 610 709 A1**

of the immersion objective is in the range of [-8.54, 20.98], that is, the third lens group G3 satisfies the following condition: $-8.54 \leq f_{32}/F_{obj} \leq 20.98$, where $f_{32}$ represents the focal length of the third lens group G3 and $f_{obj}$ represents the focal length of the immersion objective.

In an embodiment of the present disclosure, the fourth lens group G4 and the second lens group G2 are symmetrically arranged with respect to the third lens group G3. The fourth lens group G4 includes a ninth lens L9 and a tenth lens L10 sequentially cemented along the direction from the object space to the image space. An object-side surface of the ninth lens L9 is represented by S13. An image-side surface of the ninth lens L9 is cemented with an object-side surface of the tenth lens L10, and the cemented surface is represented by S14. An image-side surface of the tenth lens L10 is represented by S15. The ratio of a focal length of the fourth lens group to the focal length of the immersion objective is less than 21.10, that is, the fourth lens group G4 satisfies the following condition: $f_4/F_{obj}<21.10$, where $f_4$ represents the focal length of the fourth lens group G4 and $f_{obj}$ represents the focal length of the immersion objective.

In an embodiment of the present disclosure, the fifth lens group G5 is configured to compensate for astigmatism and field curvature and provide negative focal power. The fifth lens group G5 includes an eleventh lens L11 and a twelfth lens L12 sequentially cemented along the direction from the object space to the image space. An object-side surface of the eleventh lens L11 is represented by S16. An image-side surface of the eleventh lens L11 is cemented with an object-side surface of the twelfth lens L12, and the cemented surface is represented by S17. An image-side surface of the twelfth lens L12 is represented by S18, and a focal length of the fifth lens group is represented by $f_5$.

The immersion objective further includes an aperture stop. In an embodiment, the aperture stop is arranged between the fourth lens group G4 and the fifth lens group G5, that is, the aperture stop may be arranged between the eleventh lens L11 and the tenth lens L10. The aperture stop may further be arranged at other positions.

The fifth lens group G5 further includes a thirteenth lens L13. The thirteenth lens L13 is located on an image side of the twelfth lens L12. The thirteenth lens L13 may have negative refractive power, and may be, for example, a plano-concave lens. An object-side surface of the thirteenth lens L13 is represented by S19, and an image-side surface of the thirteenth lens L13 is represented by S20.

In an embodiment of the present disclosure, the sixth lens group G6 is configured to work in conjunction with the fifth lens group G5 to jointly control field curvature and distortion. The sixth lens group G6 includes a fourteenth lens L14 and a fifteenth lens L15 sequentially cemented along the direction from the object space to the image space. The sixth lens group G6 may be a meniscus doublet cemented lens group. An object-side surface of the fourteenth lens L14 is represented by S21. An image-side surface of the fourteenth lens L14 is cemented with an object-side surface of the fifteenth lens L15, and the cemented surface is represented by S22. An image-side surface of the fifteenth lens L15 is represented by S23. The ratio of a focal length of the sixth lens group to the focal length of the immersion objective is in the range of (-21.51,-17.4], that is, the sixth lens group G6 satisfies the following condition: $-21.51<f_6/f_{obj} \leq -17.4$, where $f_6$ represents the focal length of the sixth lens group G6 and $f_{obj}$ represents the focal length of the immersion objective. A radius of curvature $R_{21}$ of S21 satisfies the following condition: $|R_{21}/f_{obj}|<1.2$. A compound focal length $f_7$ of the fifth lens group G5 and the fourteenth lens L14 satisfy the following condition: $-6 \leq f_7/f_{obj}<-3.83$, where $f_{obj}$ represents the focal length of the immersion objective.

In an embodiment, lenses are arranged in sequence from infinity to the object space, and $F_{obj}=10mm$. A parameter detail of the immersion objective obtained by optimizations according to an embodiment is shown in table 1. In this table, spacing represents a distance, along the optical axis, between a surface and a next surface. If the two surfaces belong to a same lens, the spacing represents a central thickness of the lens. The stop plane is located on S13, with a size of 33.5mm, so as to achieve a numerical aperture of NA1.0. It may be set that: $f_1/F_{obj}=3.39$, $R_5/d_5=0.8$, $f_{32}/F_{obj}=7.27$, $f_4/f_{obj}=8.54$, $f_5/f_{obj}=-4.5$, and $f_6/f_{obj}=-21.07$.

Table 1 Parameter detail of objective lens

| Lens | | Radius of Curvature (mm) | Spacing (mm) | Refractive Index | Dispersion Coefficient |
|---|---|---|---|---|---|
| Image plane | | Infinity | Infinity | | |
| G6 | S23 | 18.825 | 9.446 | 1.85 | 23.9 |
| | S22 | 34.227 | 4.075 | 1.75 | 52.3 |
| | S21 | 10.689 | 8.450 | Air | Air |

9

(continued)

| Lens | | Radius of Curvature (mm) | Spacing (mm) | Refractive Index | Dispersion Coefficient |
|---|---|---|---|---|---|
| Image plane | | Infinity | Infinity | | |
| G5 | S20 | -12.174 | 3.537 | 1.76 | 26.5 |
| | S19 | -63.597 | 1.500 | Air | Air |
| | S18 | -22.711 | 5.480 | 1.85 | 23.9 |
| | S17 | -24.179 | 10.313 | 1.44 | 94.7 |
| | S16 | -18.627 | 0.125 | Air | Air |
| G4 | S15 | -304.508 | 9.665 | 1.43 | 95 |
| | S14 | -18.524 | 1.993 | 1.67 | 38.3 |
| | S13 | -47.714 | 0.125 | Air | Air |
| G3 | S12 | 57.291 | 10.500 | 1.49 | 85.2 |
| | S11 | -35.804 | 1.794 | 1.67 | 38.3 |
| | S10 | 163.598 | 8.000 | 1.43 | 95 |
| | S9 | -42.371 | 0.125 | Air | Air |
| G2 | S8 | 36.460 | 2.292 | 1.74 | 49.3 |
| | S7 | 24.952 | 11.215 | 1.43 | 95 |
| | S6 | -322.845 | 0.125 | Air | Air |
| L3 | S5 | 24.223 | 6.853 | 1.49 | 85.2 |
| | S4 | 70.470 | 0.125 | Air | Air |
| G1 | S3 | 17.961 | 20.669 | 1.85 | 32.3 |
| | S2 | 10.504 | 1.345 | 1.85 | 40.8 |
| | S1 | Infinity | 0.3613 (predetermined medium thickness) | 1.46 | 67.8 |
| Coverslip | | Planar | 0.66 | 1.51 | 62.5 |
| Object plane | | Planar | | | |

The immersion objective described above can implement flat-field imaging in different wavebands of an entire field of view within an object-space field of view of 1.25mm.

The diffraction-limited performance of optical components requires the measurement of wave-front aberration. It is preferred that the root-mean-square (rms) value of the wave-front aberration of the entire luminous flux is not greater than 0.07 times the tolerance wavelength. For lights with at least two different wavelengths (the wavelengths are denoted as $\lambda$), the wave-front aberration on the image information surface should not exceed $0.07\lambda$ rms within the pre-determined numerical aperture of the immersion objective imaging measurement.

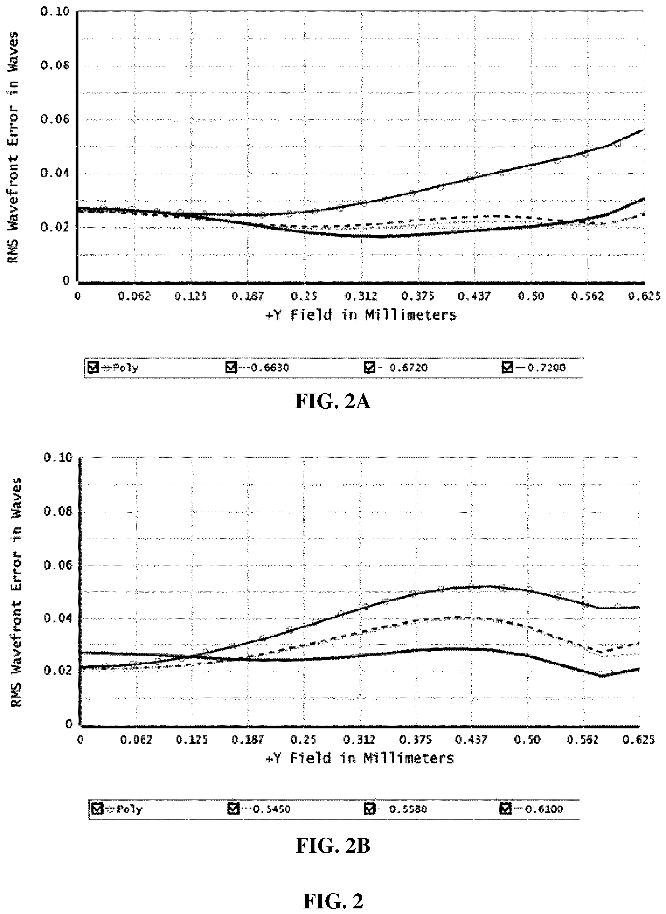

Reference is made to FIG. 2, which is a diagram of curves of wave-front error root mean square within two waveband ranges of an immersion objective according to an embodiment of the present disclosure. The horizontal axis represents a size of the object-space field of view, the vertical axis represents the wave-front error root mean-square (RMS) of the immersion objective, and curves in different colors represent curves of different wavelengths. FIG. 2A illustrates the wave-front error root mean square across the entire field of view in a red-light waveband (ranging from 0.663um to 0.72um). For red lights with wavelengths of 0.663um, 0.672um, and 0.72um, the wave-front error root mean squares within the object-space field of view are less than a diffraction limit (that is, 0.07). The Poly curve illustrates an overall image quality, that is, an image quality of a red light formed by combining lights of all wavelengths in the red-light waveband. The wave-front error root mean square within the object-space field of view of this red light is less than the diffraction limit (that is, 0.07). FIG. 2B illustrates the wave-front error root mean square across the entire field of view in a green-light waveband (ranging from 0.545um to 0.610um). For green lights with wavelengths of 0.545um, 0.558um, and 0.61um, the wave-front error root mean squares within the object-space field of view are less than the diffraction limit. The Poly curve illustrates an overall image quality; that is, an image quality of a green light formed by combining lights of all wavelengths in the green-light waveband. The wave-front error root mean square within the object-space field of view of this green light is less than the diffraction limit. It can be seen from the figure that, across the entire field of view, the immersion objective achieves diffraction-limited imaging quality over the full wavelength range of the sequencer or of the required two channels.

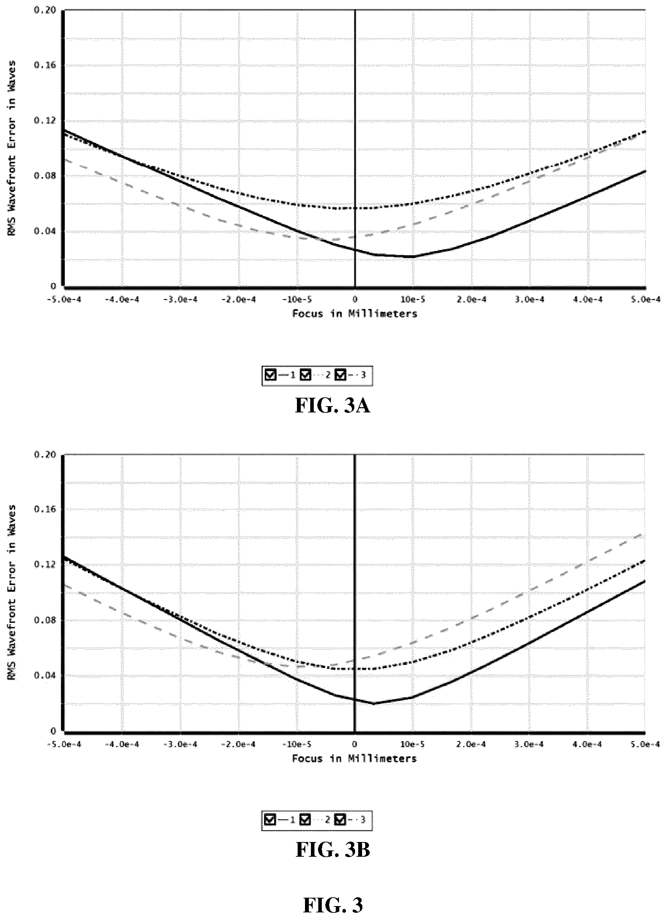

Reference is made to FIG. 3, which is a diagram of curves of wave-front errors varying with a focal plane according to an embodiment of the present disclosure. The horizontal axis represents a distance from an optimal focal plane (Focus in Millimeters), and the vertical axis represents the wave-front error root mean square (RMS Wave-front Error in Waves). FIG. 3A is a diagram of curves of wave-front errors varying with the focal plane across different fields of view in a red-light waveband (ranging from 0.663um to 0.72um). It can be seen from the figure that wave-front error RMSs are different in different fields of view. In FIG. 3A, curve 1 shows the wave-front error of an object point on the optical axis varying with the focal plane, curve 2 shows the field of view varying with the focal plane when an object height is 0.4 mm, and curve 3 shows the field of view varying with the focal plane when an object height is 0.625 mm. A field curvature across the entire field of view is less than 0.2um. FIG. 3B is a diagram of curves of wave-front errors varying with the focal plane across different fields of view in a green-light waveband (ranging from 0.545um to 0.610um). It can be seen from the figure that wave-front error RMSs are different in different fields of view. In FIG. 3B, curve 1 shows the wave-front error of an object point on the optical axis varying with the focal plane, curve 2 shows the field of view varying with the focal plane when the object height is 0.4 mm, and curve 3 shows the field of view varying with the focal plane when the object height is 0.625mm. A field curvature across the entire field of view is less than 0.2um.

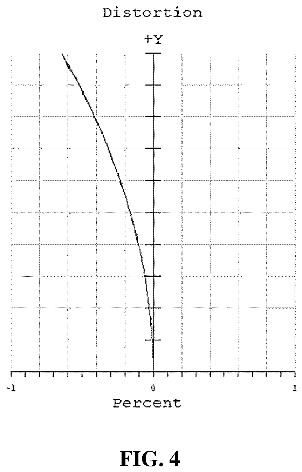

Reference is made to FIG. 4, which is a schematic diagram of a distortion feature of an immersion objective according to an embodiment of the present disclosure. The horizontal axis represents the object-space semi-field of view height (Percent), measured in mm, and the vertical axis represents distortion, measured in %. It can be seen from the figure that the distortion is less than 0.7% across the entire field of view. The immersion objective can achieve a parfocal distance of 115 mm and feature a compact structure.

In another embodiment, lenses are arranged in sequence from infinity to the object space, and $F_{obj}$=8mm. Another parameter detail of the immersion objective obtained by optimizations according to an embodiment is shown in table 2. In this table, spacing represents a distance, along the optical axis, between a surface and the next surface. If the two surfaces belong to the same lens, the spacing represents a central thickness of the lens. The stop plane is located on S15, with an aperture greater than or equal to 26.12mm, so as to achieve a numerical aperture greater than or equal to NA1.0. In an embodiment, it can be set that: $f_1/F_{obj}$=4.42, $R_5/d_5$=1, $f_{32}/F_{obj}$=6.6, $f_4/f_{obj}$=10.88, $f_5/f_{obj}$=-6.03, and $f_6/f_{obj}$=-21.51.

Table 2 Another parameter detail of objective lens

| Lens | | Radius of Curvature (mm) | Spacing (mm) | Refractive Index | Dispersion Coefficient |
|---|---|---|---|---|---|
| Image plane | | Infinity | Infinity | | |
| G6 | S23 | 15.107 | 7.581 | 1.85 | 23.9 |
| G6 | S22 | 22.149 | 3.271 | 1.75 | 52.3 |
| G6 | S21 | 8.692 | 6.781 | Air | Air |
| G5 | S20 | -9.371 | 2.839 | 1.76 | 26.5 |
| G5 | S19 | -29.847 | 0.960 | Air | Air |
| G5 | S18 | -17.749 | 4.398 | 1.85 | 23.9 |
| G5 | S17 | -19.002 | 8.276 | 1.44 | 94.7 |
| G5 | S16 | -14.896 | 0.100 | Air | Air |
| G4 | S15 | 116.153 | 7.756 | 1.43 | 95 |
| G4 | S14 | -15.289 | 1.599 | 1.67 | 38.3 |
| G4 | S13 | -54.106 | 0.100 | Air | Air |
| G3 | S12 | 46.271 | 7.277 | 1.49 | 85.2 |
| G3 | S11 | -26.713 | 1.439 | 1.67 | 38.3 |
| G3 | S10 | 96.089 | 4.718 | 1.43 | 95 |
| G3 | S9 | -41.185 | 0.100 | Air | Air |

(continued)

| Lens | | Radius of Curvature (mm) | Spacing (mm) | Refractive Index | Dispersion Coefficient |
|---|---|---|---|---|---|
| Image plane | | Infinity | Infinity | | |
| G2 | S8 | 25.904 | 1.839 | 1.74 | 49.3 |
| | S7 | 19.197 | 8.420 | 1.43 | 95 |
| | S6 | -97.297 | 0.100 | Air | Air |
| L3 | S5 | 19.743 | 5.030 | 1.49 | 85.2 |
| | S4 | 78.065 | 0.100 | Air | Air |
| G1 | S3 | 12.189 | 14.000 | 1.85 | 32.3 |
| | S2 | 4.496 | 1.080 | 1.85 | 40.8 |
| | S1 | Infinity | 0.250 (predetermined medium thickness) | 1.46 | 67.8 |
| Coverslip | | Planar | 0.480 | 1.51 | 62.5 |
| Immersion liquid | | Planar | 0.040 | 1.33 | 55.8 |
| Object plane | | Planar | | | |

The immersion objective described above has a NA=1.0, and can implement flat-field imaging in different wavebands of an entire field of view within an object-space field of view of 1mm.

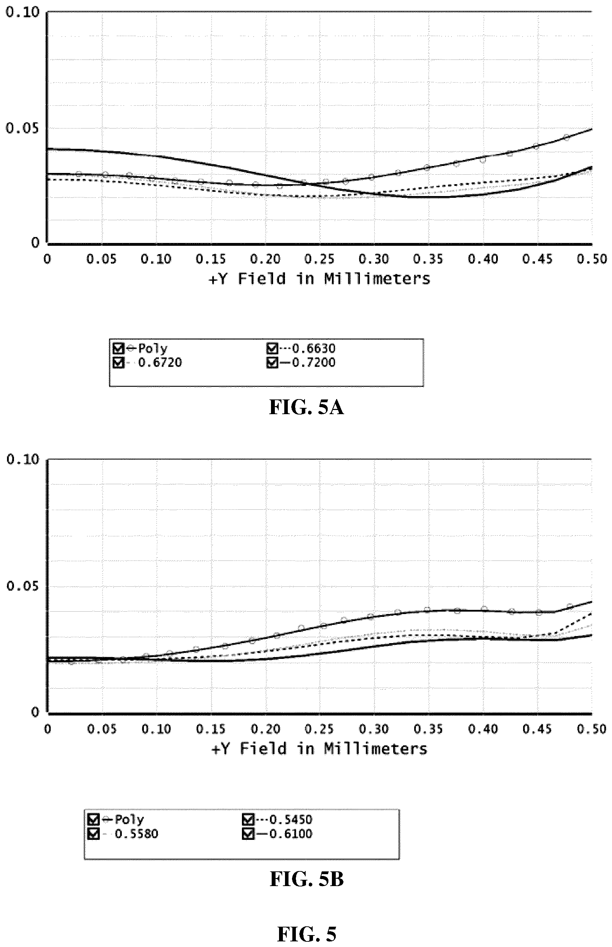

Reference is made to FIG. 5, which is a diagram of curves of wave-front error root mean squares within two waveband ranges of an immersion objective according to another embodiment of the present disclosure. The horizontal axis represents the size of the object-space field of view, the vertical axis represents the wave-front error root mean square (RMS) of the immersion objective, and curves in different colors represent curves of different wavelengths. FIG. 5A illustrates the wave-front error root mean square across the entire field of view in the red-light waveband (ranging from 0.663um to 0.72um). For red lights with wavelengths of 0.663um, 0.672um, and 0.72um, the wave-front error root mean squares within the object-space field of view are less than the diffraction limit. The Poly curve illustrates an overall image quality; that is, an image quality of a red light formed by combining lights of all wavelengths in the red-light waveband. The wave-front error root mean square within the object-space field of view of this red light is less than the diffraction limit. FIG. 5B illustrates the wave-front error root mean square across the entire field of view in the green-light waveband (ranging from 0.545um to 0.610um). For green lights with wavelengths of 0.545um, 0.558um, and 0.61um, the wave-front error root mean squares within the object-space field of view are less than the diffraction limit. The Poly curve illustrates an overall image quality; that is, an image quality of a green light formed by combining lights of all wavelengths in the green-light waveband. The wave-front error root mean square within the object-space field of view of this green light is less than the diffraction limit. It can be seen from the figure that, across the entire field of view, the immersion objective achieves diffraction-limited imaging quality over the full wavelength range of the sequencer or of the required two channels.

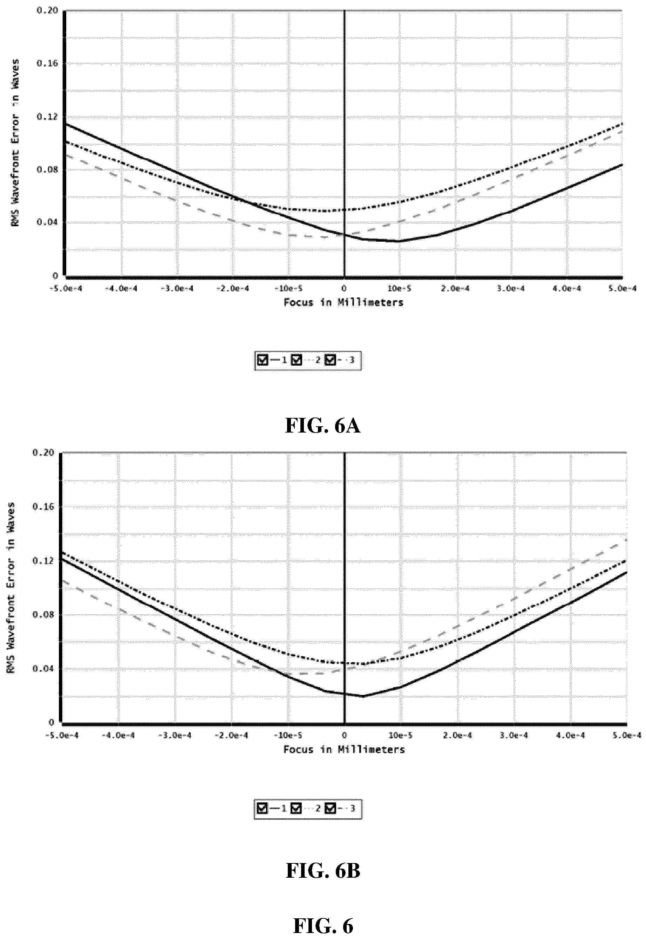

Reference is made to FIG. 6, which is a diagram of curves of wave-front errors varying with a focal plane according to another embodiment of the present disclosure. The horizontal axis represents a distance from the optimal focal plane, and the vertical axis represents the wave-front error root mean square. FIG. 3A is a diagram of curves of wave-front errors varying with the focal plane across different fields of view in the red-light waveband (ranging from 0.663um to 0.72um). It can be seen from the figure that wave-front error RMSs are different in different fields of view. In FIG. 6A, curve 1 shows the wave-front error of an object point on the optical axis varying with the focal plane, curve 2 in green shows the field of view varying with the focal plane when an object height is 0.32 mm, and curve 3 shows the field of view varying with the focal plane when an object height is 0.5 mm. A field curvature across the entire field of view is less than 0.2um. FIG. 3B is a diagram of curves of wave-front errors varying with the focal plane across different fields of view in the green-light waveband (ranging from 0.545um to 0.610um). It can be seen from the figure that wave-front error RMSs are different in different fields of view. In FIG. 6B, curve 1 shows the wave-front error of an object point on the optical axis varying with the focal plane, curve 2 shows the field of view varying with the focal plane when the object height is 0.32 mm, and curve 3 shows the field of view varying with the focal plane when the object height is 0.5 mm. The field curvature across the entire field of view is less than 0.2um.

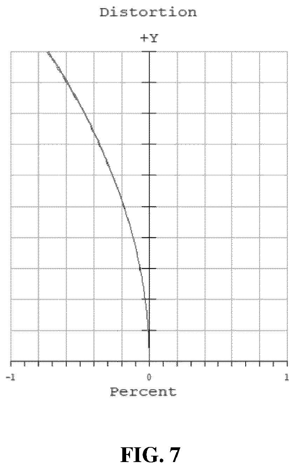

Reference is made to FIG. 7, which is a schematic diagram of a distortion feature of an immersion objective according to another embodiment of the present disclosure. The horizontal axis represents the object-space semi-field of view height, measured in mm, and the vertical axis represents the distortion, measured in %. It can be seen from the figure that the distortion is less than 0.85% across the entire field of view.

In another embodiment, lenses are arranged in sequence from infinity to the object space, and $F_{obj}$=5mm. Another parameter detail of the immersion objective obtained by optimizations according to an embodiment is shown in table 3. In this table, spacing represents a distance, along the optical axis, between a surface and a next surface. If the two surfaces belong to a same lens, the spacing represents a central thickness of the lens. The stop plane is located on S15, with an aperture=17.63mm, so as to achieve a numerical aperture of NA1.0. It may be set that: $f_1/F_{obj}$=4.26, $R_5/d_5$=0.93, $R_5/d_5$=6.914, $f_4/f_{obj}$=20.98, $f_5/f_{obj}$=-3.834, and $f_6/f_{obj}$=-17.4.

Table 3 Another parameter detail of objective lens

| Lens | | Radius of Curvature (mm) | Spacing (mm) | Refractive Index | Dispersion Coefficient |
|---|---|---|---|---|---|
| Image plane | | Infinity | Infinity | | |
| G6 | S23 | 9.577 | 4.73 | 1.85 | 23.9 |
| | S22 | 19.769 | 2.04 | 1.75 | 52.3 |
| | S21 | 5.329 | 4.23 | Air | Air |
| G5 | S20 | -5.895 | 1.77 | 1.76 | 26.5 |
| | S19 | -27.105 | 0.60 | Air | Air |
| | S18 | -12.797 | 2.74 | 1.85 | 23.9 |
| | S17 | -16.892 | 5.16 | 1.44 | 94.7 |
| | S16 | -9.727 | 0.10 | Air | Air |
| G4 | S15 | 46.422 | 4.84 | 1.43 | 95 |
| | S14 | -11.250 | 1.00 | 1.67 | 38.3 |
| | S13 | -24.776 | 0.10 | Air | Air |
| G3 | S12 | 38.296 | 4.54 | 1.49 | 85.2 |
| | S11 | -19.793 | 0.90 | 1.67 | 38.3 |
| | S10 | 33.652 | 3.30 | 1.43 | 95 |
| | S9 | -33.369 | 0.10 | Air | Air |
| G2 | S8 | 18.640 | 1.15 | 1.74 | 49.3 |
| | S7 | 13.739 | 6.00 | 1.43 | 95 |
| | S6 | -43.954 | 0.10 | Air | Air |
| L3 | S5 | 13.114 | 3.30 | 1.49 | 85.2 |
| | S4 | 55.759 | 0.10 | Air | Air |
| G1 | S3 | 8.082 | 9.00 | 1.85 | 32.3 |
| | S2 | 3.309 | 1.00 | 1.85 | 40.8 |
| | S1 | Infinity | 0.325 (predetermined medium thickness) | 1.46 | 67.8 |
| Coverslip | | Planar | 0.33 | 1.51 | 62.5 |
| Object plane | | Planar | | | |

The immersion objective described above has a NA=1.0, and can implement flat-field imaging in different wavebands of an entire field of view within an object-space field of view of 1mm.

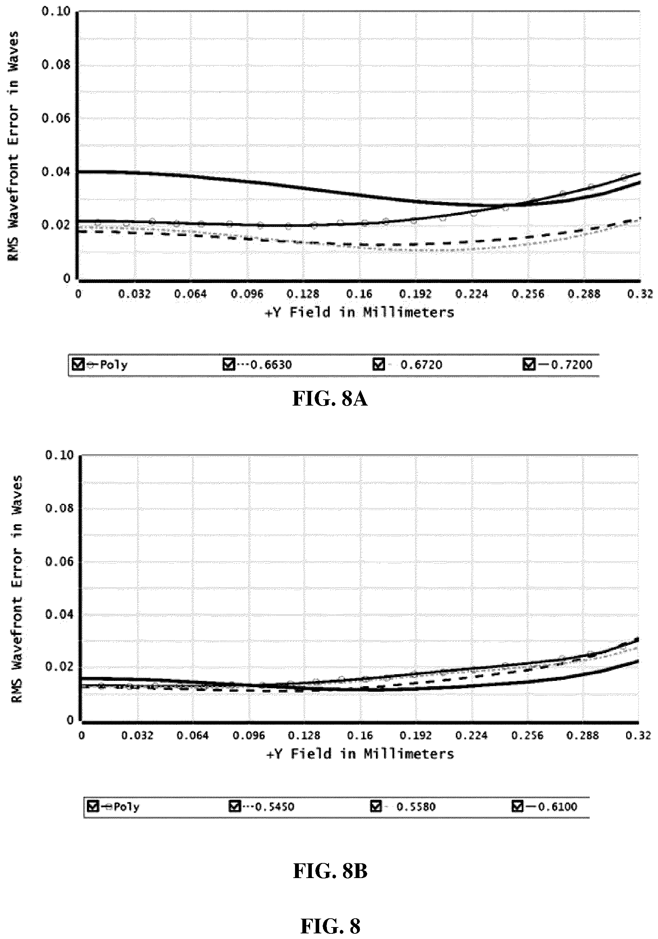

Reference is made to FIG. 8, which is a diagram of curves of wave-front error root mean squares within two waveband ranges of an immersion objective according to another embodiment of the present disclosure. The horizontal axis represents the size of the object-space field of view, the vertical axis represents the wave-front error root mean square (RMS) of the immersion objective, and curves in different colors represent curves of different wavelengths. FIG. 8A illustrates the wave-front error root mean square across the entire field of view in the red-light waveband (ranging from 0.663um to 0.72um). For red lights with wavelengths of 0.663um, 0.672um, and 0.72um, the wave-front error root mean squares within the object-space field of view are less than a diffraction limit. The Poly curve illustrates an overall image quality; that is, an image quality of a red light formed by combining lights of all wavelengths in the red-light waveband. The wave-front error root mean square within the object-space field of view of this red light is less than the diffraction limit. FIG. 8B illustrates the wave-front error root mean square across the entire field of view in the green-light waveband (ranging from 0.545um to 0.610um). For green light with wavelengths of 0.545um, 0.558um, and 0.61um, the wave-front error root mean squares within the object-space field of view that are less than the diffraction limit. The Poly curve illustrates an overall image quality; that is, an image quality of a green light formed by combining lights of all wavelengths in the green-light waveband. The wave-front error root mean square within the object-space field of view of this green light is less than the diffraction limit. It can be seen from the figure that, across the entire field of view, the immersion objective achieves diffraction-limited imaging quality over the full wavelength range of the sequencer or of the required two channels.

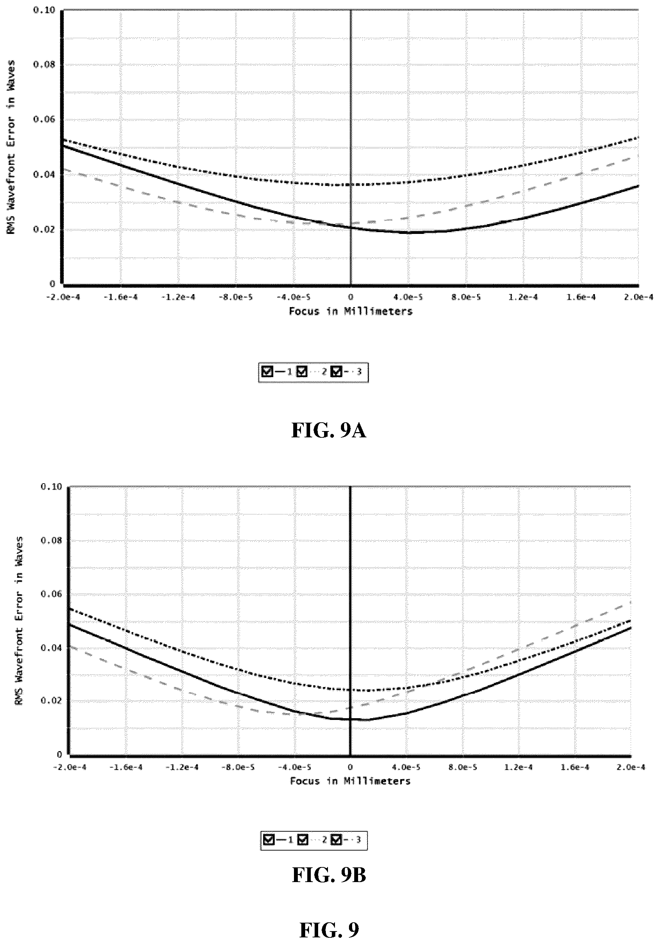

Reference is made to FIG. 9, which is a diagram of curves of wave-front errors varying with a focal plane according to another embodiment of the present disclosure. The horizontal axis represents a distance from the optimal focal plane, and the vertical axis represents the wave-front error root mean square. FIG. 3A is a diagram of curves of wave-front errors varying with the focal plane across different fields of view in the red-light waveband (ranging from 0.663um to 0.72um). It can be seen from the figure that wave-front error RMSs are different in different fields of view. In FIG. 9A, curve 1 shows the wave-front error of an object point on the optical axis varying with the focal plane, curve 2 shows the field of view varying with the focal plane when an object height is 0.2 mm, and curve 3 shows the field of view varying with the focal plane when an object height is 0.32 mm. A field curvature across the entire field of view is less than 0.2um. FIG. 3B is a diagram of curves of wave-front errors varying with the focal plane across different fields of view in the green-light waveband (ranging from 0.545um to 0.610um). It can be seen from the figure that wave-front error RMSs are different in different fields of view. In FIG.9 B, curve 1 shows the wave-front error of an object point on the optical axis varying with the focal plane, curve 2 shows the field of view varying with the focal plane when the object height is 0.2 mm, and curve 3 shows the field of view varying with the focal plane when the object height is 0.32 mm. A field curvature across the entire field of view is less than 0.05um.

The immersion objective according to embodiments of the present disclosure features a compact structure with specifications comparable to those of high-throughput microscope objectives from CG company, and achieves a reduction in length to approximately one-fourth of the length of the high-throughput microscope objectives and a reduction in weight to approximately one-tenth of the weight of the high-throughput microscope objectives, significantly minimizing both size and weight. The immersion objective with the focal length $F_{obj}$=10mm is taken as an example, the parfocal distance of the immersion objective is less than 120mm, and the weight of the weight of the immersion objective is less than 750g. The immersion objective is provided with appropriate design structure, tolerance distribution and tolerance sensitivity, making it easy to manufacture and keeping costs controllable. The immersion objective exhibits good process feasibility and has been mass-produced based on the parameters mentioned in above embodiments, achieving diffraction-limited imaging quality across the entire field of view. In addition, the immersion objective is versatile and is applicable to any microscopic system, as well as in other imaging applications requiring large fields of view and high resolution such as semiconductor front-end manufacturing and back-end inspection devices, nanotechnology, biological microscopy, and materials analysis. The immersion objective may be applicable to fast detection systems like sequencers and may also be applicable to other infinity-corrected liquid-immersion display imaging systems with the magnification ranging from 10x to 100x.

Therefore, according to the embodiments of the present disclosure, a flat-field apochromatic immersion objective with a large field of view, high resolution, NA 1.0, low distortion, and suitable for relatively thick coverslips can be realized within a relatively compact size range. The optical design of the flat-field apochromatic lens with a large field of view and a large numerical aperture is superior to the optical design of general commercial objective lenses, enabling the optical system based on the immersion objective to have excellent optical performance. For example, in the case of a sequencer, the immersion objective according to the present disclosure can improve the resolution of the optical system, increase the throughput of the sequencer, and reduce the cost of sequencing.

In a case that the to-be-detected sample includes two or more layers, the consumables cost is reduced. In an embodiment, the to-be-detected sample includes at least two detection surfaces spaced apart from each other along the optical axis of the immersion objective, for example, the to-be-detected sample is a to-be-detected chip with biomolecules adsorbed on both its upper and lower surfaces. The to-be-detected sample (to-be-detected chip) has a first detection surface and a second detection surface. Detection on the multiple-layer detection surfaces can be achieved by imaging the to-be-detected sample. However, the multi-layer chip requires the optical system to achieve clear imaging even within a focal distance range of only a few micrometers in the object space, which cannot be achieved without compensation elements for an optical system based on a large NA air objective lens. That is, when patterns are located on both the upper and lower surfaces of the sample solution, a compensation lens needs to be additionally added to the air objective lens to compensate for the spherical aberration caused by the sample solution layer, weakening the reliability of the objective lens, and failing to completely compensate the image quality to the ideal level.

Since the first medium of the immersion objective is also a liquid, which is similar to or even consistent with the sample solution regarding to the refractive index and dispersion coefficient, adjusting the working distance of the immersion medium can compensate the focal length difference between the upper and lower surfaces of the aqueous layer of the chip sample solution. In a case that the to-be-detected sample includes at least two detection surfaces spaced apart from each other along the optical axis direction of the immersion objective, for example, patterns are located on both the upper and lower surfaces of the sample solution, the thickness of the first medium is adjusted for implementing detection of different sample detection surfaces, that is, detection of the patterns on different layers. The to-be-detected sample includes a first detection surface and a second detection surface. A distance between the first detection surface and the coverslip is a first distance, and a distance between the second detection surface and the coverslip is a second distance. For detection of the first detection surface, a first medium between the first lens group and coverslip has a first thickness. For detection of the second detection surface, the first medium between the first lens group and coverslip has a second thickness.

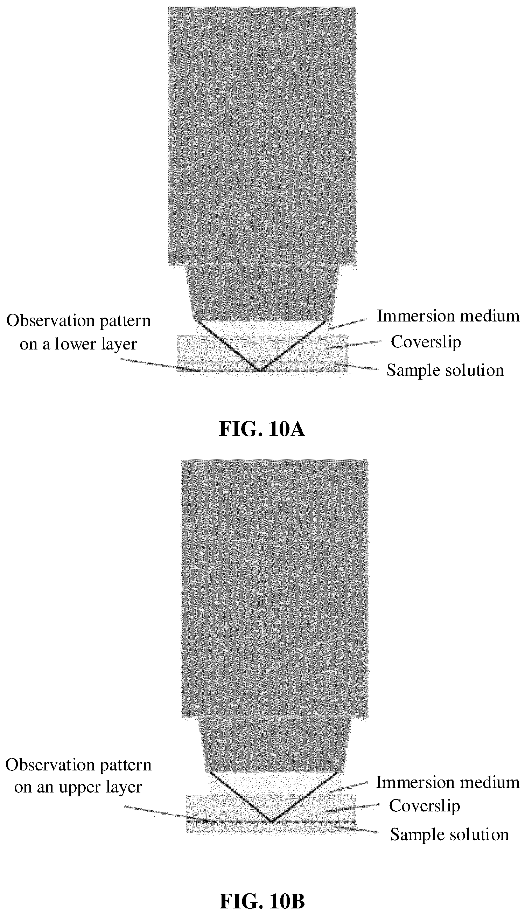

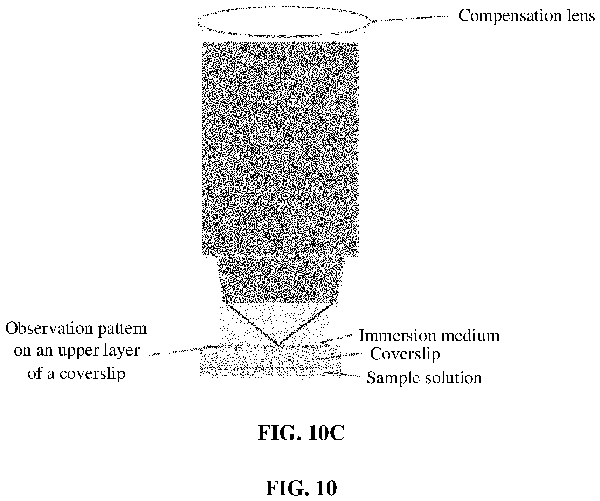

Reference is made to FIG. 10, which is a schematic diagram of operation of an immersion objective according to an embodiment of the present disclosure. As shown in FIG. 10A, the first medium with the first thickness may be configured for detection of patterns on the upper surface (serving as the first detection surface) of the sample solution. In a case that the sample solution is between the slide and the coverslip, the pattern is provided from the second detection sample fixed on the lower surface of the coverslip. As shown in FIG. 10B, the first medium with the second thickness is configured for detection of patterns on the lower surface (serving as the second detection surface) of the sample solution. In a case that the sample solution is between the slide and the coverslip, the pattern is provided from the first detection sample fixed on the upper surface of the slide.

Furthermore, when the to-be-detected sample includes multiple sample detection surfaces of the immersion objective that are spaced apart from each other along the optical axis direction, that is the sample solution has more than two layers of patterns, a compensation lens may be additionally provided for detections of more layers of patterns on the basis of adjusting the first medium. The compensation lens is removably arranged on the optical path between the immersion objective and imaging device. For example, if the first medium is provided with the to-be-detected sample, the to-be-detected sample may have a third detection surface. The third detection surface is located in the first medium on the coverslip. Thus, a compensation lens may be arranged for detection of the third detection surface. The compensation lens is located on one side of the sixth lens group away from the first lens group in the optical path. As shown in FIG. 10C, in a case that the first medium has the third thickness and is provided with a compensation lens, detection is performed on the pattern on the coverslip (as the third detection surface), so as to improve detection efficiency and reduce detection costs.

The immersion objective according to the embodiments of the present disclosure can directly focus for the detection of the dual-layer sample without additional optical elements to perform aberration compensation, and the immersion objective may further address issues related to compensation of an optical path length difference between an upper layer and a lower layer of the dual-layer chip, so as to perform detection of the dual-layer sample. Therefore, stringent tolerance requirements for the sample solution thickness are unnecessary, and the immersion objective is compatible with chips with continuously varying thicknesses of the sample solution, as long as the sum of the working distance WD and the sample solution thickness d remains constant, which simplifies detection processes.

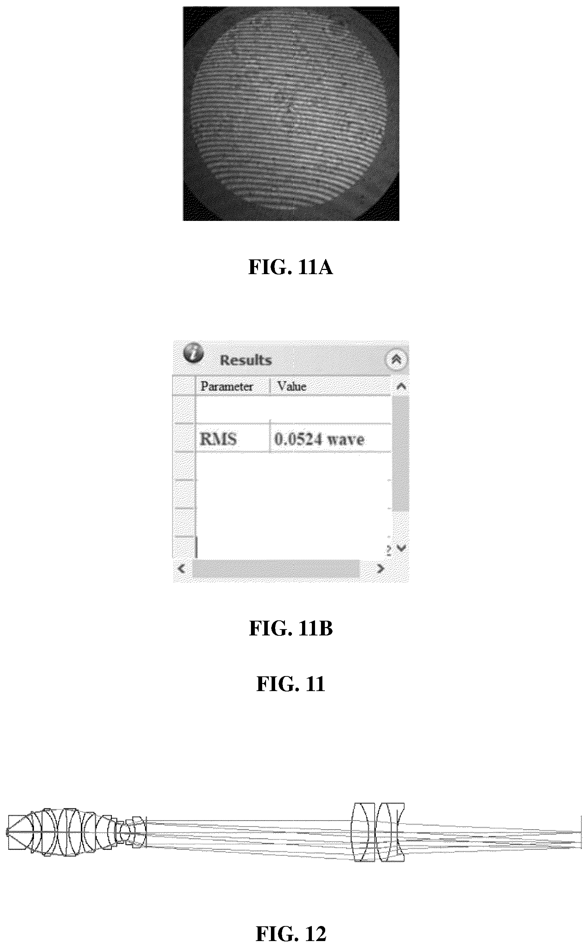

The wave-front error root mean square value of a finished product of the immersion objective can be obtained by detection via an interferometer. As shown in FIG. 11, FIG.11 illustrates a detection result of an immersion objective according to an embodiment of the present disclosure. FIG. 11A shows an interference pattern obtained through detection by the interferometer, and FIG. 11B shows the wave-front error root mean square value. The wave-front error RMS in the result is equal to 0.0524, which conforms to the design value.

According to the embodiments of the present disclosure, an immersion objective is provided. The immersion objective includes: sequentially along a direction from an object space to an image space, a first lens group with positive focal power, a third lens with positive focal power, a second lens group with positive focal power, a third lens group with positive focal power, a fourth lens group with positive focal power, a fifth lens group with negative focal power, a sixth lens group with negative focal power, and an immersion medium located on an object-side of the first lens group and immersing at least part of the first lens group. A working distance $S_0$ of the immersion medium located between the first lens group and the object space and a focal length $F_{obj}$ of the immersion objective satisfy the following conditional expression: $0<S_0/F_{obj}\leq0.16$. The combination of multiple lenses allows the immersion objective to achieve a relatively compact and lightweight design within a restricted size range, resulting in the immersion objective with a large field of view, high resolution, and minimal distortion, thus improving the optical performance of the immersion objective.

Based on the immersion objective provided according to the embodiments of the present disclosure, an optical system is further provided according to embodiments of the present disclosure. The optical system includes the immersion objective and the imaging device located on the image side of the immersion objective and working in conjunction with the immersion objective. The imaging device is configured to obtain an image of the to-be-detected sample.

Reference is made to FIG. 12, which is a schematic diagram of an optical system according to an embodiment of the present disclosure. The optical system further includes a tube lens between the immersion objective and the imaging device. The tube lens works in conjunction with the immersion objective. As shown in FIG. 12, the immersion objective is located on the left side of the tube lens. When the immersion objective is configured with tube lenses of different focal lengths, the magnifications of the high-resolution optical system are different. This means that optical modules obtained through separate magnification/diminishing of the immersion objective and tube lens, as well as the illumination system within the optical system, or the optical system obtained through collective magnification/diminishing of them, fall within the protection scope of the present disclosure. For example, the immersion objective in the first embodiment described above is configured with a tube lens with a focal length of 200mm to obtain a microscopic optical system with a magnification of 20x.

In an embodiment, the tube lens may include a structure of two doublet cemented lens groups, so as to further compensate for a lateral chromatic aberration and other residual high-order aberrations of the immersion objective. The immersion objective is parallel to the tube lens, facilitating insertion of various filters and dichroic mirrors. The obtained optical system implements the wave-front aberration of less than $0.05\lambda$ within the entire 25mm image sensor format range.

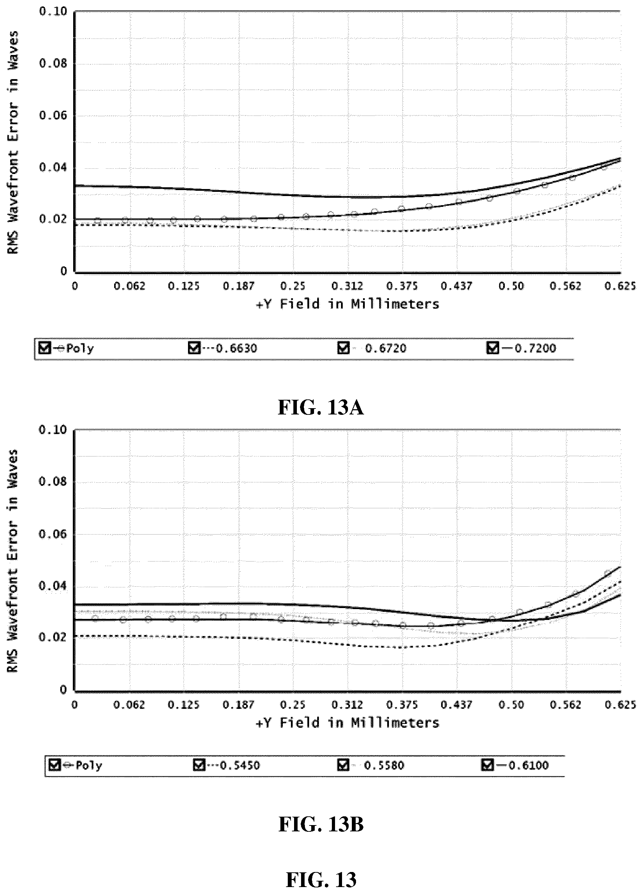

Reference is made to FIG. 13, which is a diagram of curves of wave-front error root mean square of the optical system according to an embodiment of the present disclosure. The horizontal axis represents the size of the object-space field of view, the vertical axis represents the wave-front error root mean square of the optical system, and curves in different colors represent curves of lights of different wavelengths. FIG. 13A illustrates the wave-front error root mean square of red-light channels. For red light with wavelengths of 0.663um, 0.672um, and 0.72um, the wave-front error root mean squares within the object-space field of view are less than the diffraction limit. The Poly curve illustrates an overall image quality; that is, an image quality of a red light formed by combining lights of all wavelengths in the red-light waveband. The wave-front error root mean square within the object-space field of view of this red light is less than the diffraction limit. FIG. 13B illustrates the wave-front error root mean square of green light channels. For green light with wavelengths of 0.545um, 0.558um, and 0.61um, the wave-front error root mean squares within the object-space field of view are less than the diffraction limit. The Poly curve illustrates an overall image quality; that is, an image quality of a green light formed by combining lights of all wavelengths in the green-light waveband. The wave-front error root mean square within the object-space field of view of this green light is less than the diffraction limit. It can be seen from the figure that across the entire field of view, the optical system achieves diffraction-limited imaging quality over the full wavelength range required by the sequencer.

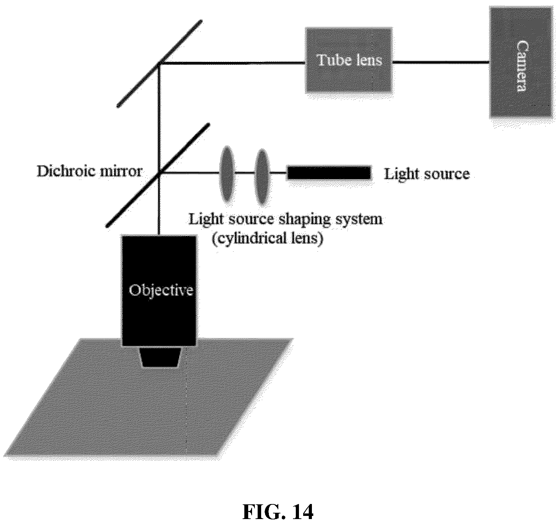

Reference is made to FIG. 14, which is a schematic structural diagram of an optical system according to another embodiment of the present disclosure. The optical system may further include a light source for stimulating samples to generate a stimulated light. The immersion objective is configured to direct a light beam generated by the light source to the sample and transmit the stimulated light. That is, the light source provides real-time illumination for the to-be-detected sample, and the immersion objective magnifies an illuminated area of the to-be-detected sample. The illumination mode may be critical illumination or Koehler illumination. In this manner, illumination shaping for area array imaging, and illumination shaping for linear array or TDI (Time Delay Integration) linear array imaging are implemented. The imaging device may further include a camera for recording the stimulated light and imaging the to-be-detected sample.

The camera may be a time delay integration (TDI) camera or another area array camera. In a case that the camera in the optical system is a time delay integration camera, the optical system may further include a light source shaping system located between the light source and the immersion objective. The light source shaping system is configured to shape the beam generated by the light source to implement line-focus shaping of light emitted by the light source. The light source shaping system may include two orthogonal cylindrical lenses.

In an embodiment of the present disclosure, the light source may include a laser optical fiber, and the optical system may further include a dichroic mirror. The dichroic mirror is configured to reflect the beam generated by the light source to the sixth lens group and transmit the beam passed through the sixth lens group. That is, the dichroic mirror may reflect a light emitted by the light source towards the immersion objective and transmit the light reflected from the to-be-detected sample and passed through the immersion objective, so that the light reaches the camera through the tube lens. A reflection mirror may further be arranged between the dichroic mirror and the tube lens and is configured to alter the beam direction.

In a case that the camera is a TDI camera, an aspect ratio of the field of view is determined by a pixel size, integration stages and a width of the image sensor of the camera and is expressed as x/y=L/D. x represents a width of the object-space field of view, y represents a linewidth of the object-side field of view, L represents the number of length-direction pixels of the image sensor of the TDI camera, and D represents the integration stages. The linewidth of the object-space field of view may be expressed as y=p*D/M, where p represents the pixel size and M represents the magnification of the imaging system.

In an embodiment of the present disclosure, the optical system further includes a compensation lens. The compensation lens is removably arranged on the optical path between the immersion objective and the imaging device.

Based on the optical system provided according to the embodiments of the present disclosure, a detection method is further provided according to embodiments of the present disclosure. The detection method includes: detecting a to-be-detected sample by using the optical system.

In an embodiment, the to-be-detected sample includes at least two sample detection surfaces spaced apart from each other along the optical axis direction of the immersion objective. The detecting a to-be-detected sample by using the optical system includes:

adjusting an immersion depth of the immersion objective in the immersion medium to detect different sample detection surfaces.

In an embodiment of the present disclosure, a nucleic acid detection system is further provided. The nucleic acid detection system includes a sequencing chip and the aforementioned optical system. The sequencing chip is configured to support the to-be-detected sample.

In an embodiment, the optical system includes an objective and an immersion medium arranged between the objective and the to-be-detected sample. An end of the objective close to the to-be-detected sample is immersed in the immersion medium, and a working distance $S_0$ of the immersion medium between the objective and the to-be-detected sample and a focal length $F_{obj}$ of the objective satisfy a conditional expression:

$$0 < S_0/F_{obj} \leq 0.16$$

The objective includes: sequentially along the direction from an object space to an image space, a first lens group with positive focal power and at least one lens group with negative focal power. The immersion medium is located on an object side of the first lens group, and at least part of the first lens group is immersed in the immersion medium.

The at least one lens group with negative focal power includes: sequentially along the direction from the object space to the image space, a third lens with positive focal power, a second lens group with positive focal power, a third lens group with positive focal power, a fourth lens group with positive focal power, a fifth lens group with negative focal power, and a sixth lens group with negative focal power.

In an embodiment, the optical system further includes an imaging device and a tube lens. The imaging device is located on an image side of the objective and works in conjunction with the objective. The tube lens is located between the objective and the imaging device.

In an embodiment, the immersion medium includes a coverslip covering the to-be-detected sample and an immersion liquid arranged on a side of the coverslip away from the to-be-detected sample. A refractive index of the immersion liquid is represented by $n_{00}$, a refractive index of the coverslip is represented by $n_{01}$, a working distance of the immersion liquid on an optical path of the objective is represented by $d_1$, and a thickness of the coverslip is represented by $d_2$, where $S_0 = d_1 + d_2$. The immersion objective further satisfies a conditional expression:

$$0 < (n_{00}d_1 + n_{01}d_2)/F_{obj} \leq 0.20$$

In an embodiment, the immersion liquid is water.

In an embodiment, the sequencing chip includes a slide spaced apart from the coverslip. The to-be-detected sample includes the first detection sample fixed to the slide and the second detection sample opposite to and spaced apart from the first detection sample and fixed to the coverslip.

According to the present disclosure, a biochemical detection method by using the optimal system described above is further provided. The biochemical detection method includes: loading a to-be-detected sample into a lane of a chip flow cell, loading a reagent with a multiple different reaction components into the lane of the chip flow cell to perform a biochemical reaction between the to-be-detected sample and the reagent, and detecting the detectable signal generated during the biochemical reaction using the optimal system described above.

The reaction components include at least one of a sample generation component and a sample analysis component.

In an embodiment, the biochemical reaction includes generating a sample in the lane of the chip flow cell, where the generating a sample in the lane of the chip flow cell includes: directing different sample generation components to flow into the lane and controlling reaction conditions in the lane to generate the sample. The biochemical reaction further includes analyzing the sample in the lane, where the analyzing the sample in the lane includes: directing the sample analysis component to flow into the lane, where the sample analysis component reacts with the sample to generate a detectable signal.

The biochemical detection method further includes: identifying the detectable signal via the optical system. The optical system includes an objective and an immersion medium arranged between the objective and the to-be-detected sample. A working distance $S_0$ of the immersion medium located between the first lens group and the object space and a focal length $F_{obj}$ of the immersion objective satisfy a conditional expression:

$$0 < S_0/F_{obj} \leq 0.16$$

In an embodiment, the objective includes: sequentially along a direction from an object space to an image space,

a first lens group with positive focal power, a third lens with positive focal power, and at least one lens group with negative focal power, and the immersion medium is located on an object side of the first lens group, and at least part of the first lens group is immersed in the immersion medium.

In an embodiment, the objective includes: sequentially along a direction from an object space to an image space: a first lens group, a third lens with positive focal power, a second lens group with positive focal power, a third lens group with positive focal power, a fourth lens group with positive focal power, a fifth lens group with negative focal power, and a sixth lens group with negative focal power.

In an embodiment, the biochemical reaction is a nucleic acid sequencing reaction and the to-be-detected sample is a nucleic acid sequencing library.

In an embodiment, the detectable signal is an optical signal. In other embodiments, the detectable signal may further include a signal other than the optical signal, and detection of the detectable signal may be performed by identification using a system other than the optical system.

In an embodiment, the to-be-detected sample is a tissue sample and the biochemical reaction is a specific binding reaction.

During detection of the detectable signal by using the optical system described above, image quality can be adjusted by adjusting the immersion depth of the objective in the immersion medium. In an embodiment, the biochemical reaction is a nucleic acid sequencing reaction and the to-be-detected sample is a nucleic acid sequencing library. The above-described chip flow cell may have, for example, the structure of the above-described multi-layer to-be-detected sample. By adjusting the immersion depth of the objective lens in the immersion medium, the detection of nucleic acid sequencing libraries on different detection surfaces can be implemented. In an embodiment, the detectable signal is an optical signal, the to-be-detected sample may further be the tissue sample, and the biochemical reaction may be a specific binding reaction.

When elements in various embodiments of the present disclosure are illustrated, the articles "a," "an", "this", and "the" are intended to indicate that the number of such element(s) is one or more. The terms "comprise", "include", and "have" are all inclusive terms and indicate that there may be an additional element besides listed elements.

The embodiments in the present disclosure are described in a progressive manner, the same and similar parts of the various embodiments in this specification can be referred to each other, and each embodiment focuses on the differences from other embodiments. For example, the embodiments of the detection method, the nucleic acid detection system, and the biochemical detection method can refer to the description of embodiments of the immersion objective.

The above description only represents the preferred implementation modes of the present disclosure. Although the present disclosure has been disclosed as above in preferred embodiments, it is not intended to limit the present disclosure. With the method and technical content disclosed above, those skilled in the art can make some variations and improvements to the technical solutions of the present disclosure, or make some equivalent variations on the embodiments without departing from the scope of technical solutions of the present disclosure. All simple modifications, equivalent variations and improvements made based on the technical essence of the present disclosure without departing the content of the technical solutions of the present disclosure fall within the protection scope of the technical solutions of the present disclosure.

**Claims**

1. An immersion objective, comprising: sequentially along a direction from an object space to an image space, a first lens group with positive focal power, at least one lens group with negative focal power and an immersion medium, wherein the immersion medium is located on an object side of the first lens group, at least part of the first lens group is immersed in the immersion medium, and a working distance $S_0$ of the immersion medium located between the first lens group and the object space and a focal length $F_{obj}$ of the immersion objective satisfy a conditional expression:

$$0 < S_0/F_{obj} \leq 0.16$$

2. The immersion objective according to claim 1, wherein the at least one lens group with negative focal power comprises: sequentially along the direction from the object space to the image space, a third lens with positive focal power, a second lens group with positive focal power, a third lens group with positive focal power, a fourth lens group with positive focal power, a fifth lens group with negative focal power and a sixth lens group with negative focal power.

3. The immersion objective according to any one of claims 1 and 2, wherein the immersion medium comprises a first medium with a first refractive index $n_{00}$ and a second medium with a second refractive index $n_{01}$, the first medium is located between the second medium and the first lens group and has a thickness $d_1$, and the second medium has a

thickness $d_2$, where $S_0 = d_1 + d_2$, and the immersion objective further satisfies a conditional expression:

$$0 < (n_{00}d_1 + n_{01}d_2)/F_{obj} \leq 0.20$$

**4.** The immersion objective according to claim 3, wherein $n_{01} > n_{00}$.

**5.** The immersion objective according to claim 4, wherein the first medium is a liquid and the second medium is a solid.

**6.** The immersion objective according to claim 5, wherein the first medium is water and the second medium is a glass plate.

**7.** The immersion objective according to claim 2, wherein the first lens group, the second lens group, the fourth lens group, and the sixth lens group are doublet cemented lens groups, the fifth lens group comprises a doublet cemented lens group, and the second lens group and the fourth lens group are symmetrically arranged with respect to the third lens group.

**8.** The immersion objective according to claim 7, wherein the first lens group comprises a first lens and a second lens that are sequentially cemented along the direction from the object space to the image space, an object-side surface of the first lens is a plane surface, an image-side surface of the first lens is a convex surface, an image-side surface of the second lens is a convex surface, and a ratio of a focal length of the first lens group to the focal length of the immersion objective is in a range of [3.39, 4.4].

**9.** The immersion objective according to claim 7, wherein the third lens is a meniscus lens, a ratio of a radius of curvature of an image-side surface of the third lens to a first distance is in a range of (-0.8, 1.0], the first distance is an axial distance between the image-side surface of the third lens and an object plane, and a ratio of a focal length of the third lens and the focal length of the immersion objective is in a range of (-6.6, 7.27].

**10.** The immersion objective according to claim 7, wherein the second lens group comprises a fourth lens and a fifth lens that are sequentially cemented along the direction from the object space to the image space, and an absolute value of a difference between a refractive index of the fourth lens and a refractive index of the fifth lens is greater than 0.2.

**11.** The immersion objective according to claim 7, wherein the third lens group is a triplet cemented lens group, and the third lens group comprises a sixth lens, a seventh lens, and an eighth lens that are sequentially cemented along the direction from the object space to the image space.

**12.** The immersion objective according to claim 7, wherein the fourth lens group comprises a ninth lens and a tenth lens that are sequentially cemented along the direction from the object space to the image space, and a ratio of a focal length of the fourth lens group to the focal length of the immersion objective is less than 21.10.

**13.** The immersion objective according to claim 7, further comprising an aperture stop.

**14.** The immersion objective according to claim 7, wherein the sixth lens group comprises a fourteenth lens and a fifteenth lens that are sequentially cemented along the direction from the object space to the image space, and an ratio of a focal length of the sixth lens group to the focal length of the immersion objective is in a range of (-21.51, -17.4].

**15.** The immersion objective according to claim 7, wherein the doublet cemented lens group comprised in the fifth lens group comprises an eleventh lens and a twelfth lens that are sequentially cemented along the direction from the object space to the image space, the fifth lens further comprises a thirteenth lens, and the thirteenth lens is located between the twelfth lens and the sixth lens group.

**16.** An optical system, comprising the immersion objective according to any one of claims 1 to 15 and an imaging device, wherein the imaging device is located on an image side of the immersion objective and works in conjunction with the immersion objective.

**17.** The optical system according to claim 16, further comprising a tube lens located between the immersion objective and the imaging device.

**18.** The optical system according to claim 16 or claim 17, further comprising a light source for stimulating a to-be-detected sample to generate a stimulated light, wherein the immersion objective is configured to direct a light beam generated by the light source to the to-be-detected sample and transmit the stimulated light, and the imaging device comprises a time delay integration camera, wherein the time delay integration camera is configured to record the stimulated light and image the to-be-detected sample.

**19.** The optical system according to claim 18, further comprising a light source shaping system located between the light source and the immersion objective, wherein the light source shaping system is configured to shape the light beam generated by the light source.

**20.** The optical system according to claim 16 or 17, further comprising a compensation lens, wherein the compensation lens is removably arranged at an optical path between the immersion objective and the imaging device.

**21.** A detection method, comprising:
detecting a to-be-detected sample by using the optical system according to any one of claims 16 to 20.

**22.** The detection method according to claim 21, wherein the to-be-detected sample comprises at least two sample detection surfaces spaced apart from each other along an optical axis direction of the immersion objective, wherein the detecting a to-be-detected sample by using the optical system comprises:
adjusting an immersion depth of the immersion objective in the immersion medium to detect different sample detection surfaces.

**23.** A nucleic acid detection system, comprising a sequencing chip and an optical system, wherein the sequencing chip is configured to support a to-be-detected sample, and the optical system comprises an objective and an immersion medium arranged between the objective and the to-be-detected sample, and wherein an end of the objective close to the to-be-detected sample is immersed in the immersion medium, and a working distance $S_0$ of the immersion medium between the objective and the to-be-detected sample and a focal length $F_{obj}$ of the objective satisfy a conditional expression:

$$0 < S_0/F_{obj} \leq 0.16$$

**24.** The nucleic acid detection system according to claim 23, wherein the objective comprises, sequentially along a direction from an object space to an image space, a first lens group with positive focal power and at least one lens group with negative focal power, wherein the immersion medium is located on an object side of the first lens group and at least part of the first lens group is immersed in the immersion medium.

**25.** The nucleic acid detection system according to claim 24, wherein the at least one lens group with negative focal power comprises: sequentially along the direction from the object space to the image space, a third lens with positive focal power, a second lens group with positive focal power, a third lens group with positive focal power, a fourth lens group with positive focal power, a fifth lens group with negative focal power, and a sixth lens group with negative focal power.

**26.** The nucleic acid detection system according to claim 24, wherein the optical system further comprises:

an imaging device, located on an image side of the objective and works in conjunction with the objective; and
a tube lens, located between the objective and the imaging device.

**27.** The nucleic acid detection system according to claim 24, wherein the immersion medium comprises a coverslip covering the to-be-detected sample and an immersion liquid arranged on a side of the coverslip away from the to-be-detected sample; a refractive index of the immersion liquid is represented by $n_{00}$, a refractive index of the coverslip is represented by $n_{01}$, a working distance of the immersion liquid on an optical path of the objective is represented by $d_1$, and a thickness of the coverslip is represented by $d_2$, where $S_0 = d_1 + d_2$; and the immersion objective further satisfies a conditional expression:

$$0 < (n_{00}d_1 + n_{01}d_2)/F_{obj} \leq 0.20$$

**28.** The nucleic acid detection system according to claim 24, wherein the immersion liquid is water.

**29.** The nucleic acid detection system according to claim 24, wherein the sequencing chip comprises a slide spaced apart from a coverslip, and the to-be-detected sample comprises a first detection sample fixed to the slide and a second detection sample opposite to and spaced apart from the first detection sample and fixed to the coverslip.

**30.** A biochemical detection method, comprising:

loading a to-be-detected sample into a lane of a chip flow cell; and
loading a reagent with a plurality of different reaction components into the lane of the chip flow cell to perform a biochemical reaction between the to-be-detected sample and the reagent, wherein
the reaction components comprise at least one of: a sample generation component and a sample analysis component;
optionally, the biochemical reaction comprises generating a sample in the lane of the chip flow cell, which comprises: directing different sample generation components to flow into the lane and controlling reaction conditions in the lane to generate the sample; and
the biochemical reaction comprises analyzing the sample in the lane, which comprises: directing the sample analysis component to flow into the lane, wherein the sample analysis component reacts with the sample to generate a detectable signal,
wherein the biochemical detection method further comprises:
identifying the detectable signal using an optical system, wherein the optical system comprises an objective and an immersion medium arranged between the objective and the to-be-detected sample, and a working distance $S_0$ of the immersion medium located between the objective and the to-be-detected sample and a focal length $F_{obj}$ of the objective satisfy a conditional expression:

$$0 < S_0/F_{obj} \leq 0.16$$

**31.** The biochemical detection method according to claim 30, wherein the objective comprises: sequentially along a direction from an object space to an image space, a first lens group with positive focal power, a third lens with positive focal power, and at least one lens group with negative focal power, and the immersion medium is located on an object side of the first lens group, and at least part of the first lens group is immersed in the immersion medium.

**32.** The biochemical detection method according to claim 30, wherein the objective comprises: sequentially along a direction from an object side to an image side: a first lens group, a third lens with positive focal power, a second lens group with positive focal power, a third lens group with positive focal power, a fourth lens group with positive focal power, a fifth lens group with negative focal power, and a sixth lens group with negative focal power.

**33.** The biochemical detection method according to claim 31, wherein the biochemical reaction is a nucleic acid sequencing reaction and the to-be-detected sample is a nucleic acid sequencing library.

**33.** The biochemical detection method according to claim 30, wherein the detectable signal is an optical signal.

**34.** The biochemical detection method according to claim 33, wherein the to-be-detected sample is a tissue sample and the biochemical reaction is a specific binding reaction.

Gx

G1

m

S

**FIG. 1A**

Gxn

Gx1

G1

m

S

**FIG. 1B**

Gx

G1

m

S

**FIG. 1C**

Gx

G1

m

S

**FIG. 1D**

G6  G5  G4  G3  G2  G1

L15 L14 L13 L12 L11 L10 L9 L8 L7 L6 L5 L4 L3 L2 L1

S23 S22 S21 S20 S19 S18 S17 S16 S15 S14 S13 S12 S11 S10 S9 S8 S7 S6 S5 S4 S3 S2 S1

**FIG. 1E**

FIG. 2A

FIG. 2B

FIG. 2

**FIG. 3A**

**FIG. 3B**

**FIG. 3**

Distortion

FIG. 4

| ☑↩Poly | ☑···0.6630 |
| ☑· ·0.6720 | ☑—0.7200 |

**FIG. 5A**

| ☑↩Poly | ☑···0.5450 |
| ☑· ·0.5580 | ☑—0.6100 |

**FIG. 5B**

**FIG. 5**

**FIG. 6A**

**FIG. 6B**

**FIG. 6**

Distortion

**FIG. 7**

**FIG. 8A**

**FIG. 8B**

**FIG. 8**

30

**FIG. 9A**

**FIG. 9B**

**FIG. 9**

Observation pattern
on a lower layer

Immersion medium
Coverslip
Sample solution

**FIG. 10A**

Observation pattern
on an upper layer

Immersion medium
Coverslip
Sample solution

**FIG. 10B**

Compensation lens

Observation pattern
on an upper layer
of a coverslip

Immersion medium
Coverslip
Sample solution

**FIG. 10C**

**FIG. 10**

**FIG. 11A**

**FIG. 11B**

**FIG. 11**

**FIG. 12**

**FIG. 13A**

**FIG. 13B**

**FIG. 13**

**FIG. 14**

## INTERNATIONAL SEARCH REPORT

| International application No. |
|---|
| **PCT/CN2022/127815** |

| A. | CLASSIFICATION OF SUBJECT MATTER |
|---|---|

G02B21/02(2006.01)i;  C12Q1/6809(2018.01)i;  C12Q1/6869(2018.01)i

According to International Patent Classification (IPC) or to both national classification and IPC

| B. | FIELDS SEARCHED |
|---|---|

Minimum documentation searched (classification system followed by classification symbols)

IPC:G02B21; C12Q1

Documentation searched other than minimum documentation to the extent that such documents are included in the fields searched

Electronic data base consulted during the international search (name of data base and, where practicable, search terms used)

CNTXT; ENTXTC: 浸, 透镜组, 正, 负, 工作距离, 焦距, 核酸, DNA, RNA, 芯片, immers+, lens group, positive, negative, working distance, focal distance, nucleic acid, chip?

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | US 2002089760 A1 (NIKON CORP.) 11 July 2002 (2002-07-11) description, paragraphs [0002]-[0071], and figures 1-7 | 1-22, 24, 25, 28, 31, 32 |
| Y | CN 105861293 A (DIRECT GENOMICS BIOTECHNOLOGY CO., LTD.) 17 August 2016 (2016-08-17) description, paragraphs [0002]-[0133], and figures 1-16 | 23-34 |
| Y | CN 113840924 A (ELEMENT BIOSCIENCES, INC.) 24 December 2021 (2021-12-24) description, paragraphs [0002]-[0484], and figures 1-44B | 1-34 |
| Y | US 2011063735 A1 (YAMAGUCHI KOTARO) 17 March 2011 (2011-03-17) description, paragraphs [0002]-[0061], and figures 1-7 | 1-22, 24, 25, 28, 31, 32 |
| Y | JP 2003015047 A (NIKON CORP.) 15 January 2003 (2003-01-15) description, paragraphs [0005]-[0043], and figures 1-9 | 1-22, 24, 25, 28, 31, 32 |
| A | CN 112526737 A (MOTIC CHINA GROUP CO., LTD.) 19 March 2021 (2021-03-19) entire document | 1-34 |
| A | US 2016116724 A1 (OLYMPUS CORP.) 28 April 2016 (2016-04-28) entire document | 1-34 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **20 July 2023** | **21 July 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| International application No. |
| --- |
| **PCT/CN2022/127815** |

| Patent document cited in search report | | | Publication date (day/month/year) | Patent family member(s) | | | Publication date (day/month/year) |
| --- | --- | --- | --- | --- | --- | --- | --- |
| US | 2002089760 | A1 | 11 July 2002 | US | 6519092 | B2 | 11 February 2003 |
| | | | | JP | 2002148519 | A | 22 May 2002 |
| | | | | JP | 4692698 | B2 | 01 June 2011 |
| CN | 105861293 | A | 17 August 2016 | US | 2018135119 | A1 | 17 May 2018 |
| | | | | US | 10301676 | B2 | 28 May 2019 |
| | | | | WO | 2017174041 | A1 | 12 October 2017 |
| CN | 113840924 | A | 24 December 2021 | EP | 4056988 | A1 | 14 September 2022 |
| | | | | AU | 2022204320 | A1 | 07 July 2022 |
| | | | | GB | 202115320 | D0 | 08 December 2021 |
| | | | | GB | 2598498 | A | 02 March 2022 |
| | | | | GB | 2598498 | B | 19 October 2022 |
| | | | | US | 2022251644 | A1 | 11 August 2022 |
| | | | | KR | 20210154868 | A | 21 December 2021 |
| | | | | KR | 102538825 | B1 | 31 May 2023 |
| | | | | EP | 3942039 | A1 | 26 January 2022 |
| | | | | EP | 3942039 | A4 | 18 May 2022 |
| | | | | DE | 202021001858 | U1 | 05 August 2021 |
| | | | | AU | 2021207987 | A1 | 18 November 2021 |
| | | | | AU | 2021207987 | B2 | 24 March 2022 |
| | | | | US | 2021223161 | A1 | 22 July 2021 |
| | | | | US | 11408032 | B2 | 09 August 2022 |
| | | | | US | 2021333210 | A1 | 28 October 2021 |
| | | | | US | 11261489 | B2 | 01 March 2022 |
| | | | | DE | 112021000050 | T5 | 25 May 2022 |
| | | | | GB | 2604297 | A | 31 August 2022 |
| | | | | GB | 2604297 | B | 21 June 2023 |
| | | | | CA | 3137050 | A1 | 22 July 2021 |
| | | | | US | 2021332416 | A1 | 28 October 2021 |
| | | | | US | 11365444 | B2 | 21 June 2022 |
| | | | | EP | 3988922 | A1 | 27 April 2022 |
| | | | | IL | 287375 | A | 01 December 2021 |
| | | | | EP | 4060321 | A1 | 21 September 2022 |
| | | | | EP | 4089182 | A1 | 16 November 2022 |
| | | | | AU | 2021254575 | A1 | 18 November 2021 |
| | | | | IL | 287430 | A | 01 December 2021 |
| | | | | GB | 202306944 | D0 | 28 June 2023 |
| | | | | US | 2022025457 | A1 | 27 January 2022 |
| | | | | US | 11339433 | B2 | 24 May 2022 |
| | | | | KR | 20220118300 | A | 25 August 2022 |
| | | | | US | 2022136047 | A1 | 05 May 2022 |
| | | | | WO | 2021146597 | A1 | 22 July 2021 |
| | | | | SG | 10202112013 | QA | 30 December 2021 |
| | | | | US | 2022251643 | A1 | 11 August 2022 |
| | | | | US | 2022267842 | A1 | 25 August 2022 |
| | | | | US | 11053540 | B1 | 06 July 2021 |
| | | | | US | 2021222238 | A1 | 22 July 2021 |
| | | | | GB | 202116314 | D0 | 29 December 2021 |
| | | | | GB | 2598233 | A | 23 February 2022 |
| | | | | GB | 2598233 | B | 16 November 2022 |
| | | | | US | 11060138 | B1 | 13 July 2021 |

Form PCT/ISA/210 (patent family annex) (July 2022)

## INTERNATIONAL SEARCH REPORT
### Information on patent family members

| International application No. |
| --- |
| **PCT/CN2022/127815** |

| Patent document cited in search report | | | Publication date (day/month/year) | Patent family member(s) | | | Publication date (day/month/year) |
| --- | --- | --- | --- | --- | --- | --- | --- |
| | | | | US | 2021222239 | A1 | 22 July 2021 |
| | | | | US | 2021333211 | A1 | 28 October 2021 |
| | | | | US | 11459608 | B2 | 04 October 2022 |
| | | | | JP | 2022540970 | A | 21 September 2022 |
| | | | | JP | 7223165 | B2 | 15 February 2023 |
| | | | | GB | 202306947 | D0 | 28 June 2023 |
| | | | | GB | 202207966 | D0 | 13 July 2022 |
| | | | | GB | 2605310 | A | 28 September 2022 |
| | | | | GB | 2605310 | B | 24 May 2023 |
| | | | | JP | 2022129394 | A | 05 September 2022 |
| | | | | GB | 2604072 | A | 24 August 2022 |
| | | | | GB | 2604072 | B | 21 June 2023 |
| | | | | SG | 11202111573 | VA | 29 November 2021 |
| US | 2011063735 | A1 | 17 March 2011 | EP | 2192434 | A1 | 02 June 2010 |
| | | | | EP | 2192434 | A4 | 15 December 2010 |
| | | | | EP | 2192434 | B1 | 05 August 2020 |
| | | | | US | 8199408 | B2 | 12 June 2012 |
| | | | | WO | 2009035072 | A1 | 19 March 2009 |
| | | | | JPWO | 2009035072 | A1 | 24 December 2010 |
| | | | | JP | 4863132 | B2 | 25 January 2012 |
| JP | 2003015047 | A | 15 January 2003 | US | 2003043473 | A1 | 06 March 2003 |
| CN | 112526737 | A | 19 March 2021 | | None | | |
| US | 2016116724 | A1 | 28 April 2016 | JP | 2016085335 | A | 19 May 2016 |
| | | | | JP | 6367685 | B2 | 01 August 2018 |
| | | | | US | 2017322404 | A1 | 09 November 2017 |
| | | | | US | 9746658 | B2 | 29 August 2017 |

Form PCT/ISA/210 (patent family annex) (July 2022)